# EUROPEAN PATENT SPECIFICATION

(11) **EP 4 479 556 B1**
(45) Date of publication and mention of the grant of the patent: **04.03.2026**
(21) Application number: 23711345.1
(22) Date of filing: 13.02.2023
(51) Int. Cl.: C12Q 1/6844

(54) **INTERNAL CONTROLS FOR NUCLEIC ACID AMPLIFICATION**
INTERNE KONTROLLEN FÜR NUKLEINSÄUREAMPLIFIKATION
TÉMOINS INTERNES POUR AMPLIFICATION D'ACIDE NUCLÉIQUE

(30) Priority: 14.02.2022 US 202263309827 P
(43) Date of publication of application: 25.12.2024
(73) Proprietor: Becton, Dickinson and Company, Franklin Lakes, NJ 07417-1880 (US)
(72) Inventor: RICHART, Gregory A., San Diego, California 92121 (US); TOLA, Sydney Bryce, San Diego, California 92121 (US); TYLER, Ejan Marie, San Diego, California 92121 (US); ZHANG, Honghua, San Diego, California 92121 (US)
(74) Representative: Carpmaels & Ransford LLP
(86) International application number: PCT/US2023/062485
(87) International publication number: WO 2023/154920

(56) References cited:
- WO-A1-2019/163064
- LEGGATE JOHANNA ET AL: "An Internal Amplification Control System Based on Primer-Dimer Formation for PCR Product Detection by DNA Hybridization", JOURNAL OF FOOD PROTECTION, vol. 69, no. 9, 1 September 2006 (2006-09-01), pages 2280 - 2284, XP055903831, Retrieved from the Internet <URL:https://meridian.allenpress.com/jfp/article/69/9/2280/171449/An-Internal-Amplification-Control-System-Based-on>
- MARY KATHERINE JOHANSSON ED - VLADIMIR V DIDENKO (ED): "Choosing Reporter-Quencher Pairs for Efficient Quenching Through Formation of Intramolecular Dimers", 1 January 2006, FLUORESCENT ENERGY TRANSFER NUCLEIC ACID PROBES : DESIGNS AND PROTOCOLS; IN: METHODS IN MOLECULAR BIOLOGY; ISSN 1064-3745; VOL. 1045; FLUORESCENT ENERGY TRANSFER NUCLEIC ACID PROBES : DESIGNS AND PROTOCOLS; [METHODS IN MOLECULAR BIOLOGY; ISSN 1064-37, ISBN: 978-1-58829-380-0, XP008170702
- ROHAN T. RANASINGHE ET AL: "Fluorescence based strategies for genetic analysis", CHEMICAL COMMUNICATIONS, no. 44, 1 January 2005 (2005-01-01), pages 5487, XP055009074, ISSN: 1359-7345, DOI: 10.1039/b509522k

## Description

### REFERENCE TO SEQUENCE LISTING

The present application is being filed along with a Sequence Listing in electronic format. The Sequence Listing is provided as a file entitled 68EB-317346-WO, created February 13, 2023, which is 8.0 kilobytes in size.

### BACKGROUND

### Field

The present disclosure relates generally to molecular biology. More specifically, disclosed herein include methods and compositions for monitoring nucleic acid amplifications.

### Description of the Related Art

Two main strategies have been employed for internal control ("IC") in a nucleic acid amplification assay: competitive and noncompetitive internal control approaches. The main difference between the two strategies is whether the internal control component shares one common set of primers for internal control and target amplification.

Using the competitive IC strategy, there is always some competition between target and IC with the simultaneous amplification of target and IC fragments flanked by the same primers. The competition by IC amplification can lower target amplification efficiency and thereby result in a lower detection limit. Competitive IC methods therefore require more optimization on IC to achieve a sensitive detection limit. In a noncompetitive approach, the target and IC are amplified using a different primer set for each. Although the kinetics of each reaction are not influenced by a competition for the primers, the IC amplification must be limited by a controlled concentration of the IC specific primers and/or IC template to limit the competition between the target and the IC reactions for primers and DNA polymerase. Therefore, nucleotide composition, copy number, and size of the IC must be carefully considered.

There is a need for overcoming the challenges presented by competition of target amplifications in the current competitive and noncompetitive IC methods, and reducing complexity in performing quality control over nucleic acid amplification. WO2019163064A1 describes a method for measuring success or failure of PCR.

### SUMMARY

The present invention is set out in the appended set of claims. The terminologies "embodiment" and "embodiments" are to be construed as embodiment(s) of the invention only in as far as they fall within the scope of the appended claims. Otherwise, they refer to embodiments of the disclosure only, for reference purposes. Disclosed herein include methods for monitoring an amplification reaction. In some embodiments, the method comprises: (a) providing a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label; (b) contacting the first quality control primer and the second quality control primer, thereby forming a duplex via hybridization between the 3' overlapping regions of the first and second quality control primers; (c) subjecting the duplex to an amplification condition, thereby generating an extended duplex; and (d) detecting a signal generated from the quenchable label of the first quality control primer during the amplification reaction to determine generation of the extended duplex, wherein a decrease in the signal during the amplification reaction indicates generation of the extended duplex.

The quenchable label can be a fluorophore. The location of the quenchable on a quality control primer can vary. In some embodiments, the quenchable label is outside of the 3' overlapping region of the first quality control primer. In some embodiments, the quenchable label is at 5' terminus of the first quality control primer. In some embodiments, the quenchable label is in the 3' overlapping region of the first quality control primer.

The second quality control primer can comprise a quencher. In some embodiments, the quencher is outside of the 3' overlapping region of the second quality control primer. In some embodiments, the quencher is at 5' terminus of the second quality control primer. In some embodiments, the quencher is in the 3' overlapping region of the second quality control primer. In some embodiments, the second quality control primer does not comprise a quencher. In some embodiments, neither of the first and second quality control primers comprises a quencher.

The first quality control primer, the second quality control primer, or both, can comprise one or more modified nucleotides. For example, each of the 3' overlapping regions of the first and second quality control primers can comprise one or more modified nucleotides. In some embodiments, the one or more modified nucleotides comprise a spacer, an a-basic site, an un-methylated RNA base, a 2'-O-methylated nucleotide, and any combination thereof. In some embodiments, at least one of the one or more modified nucleotides is a 2'-O-methylated nucleotide. In some embodiments, the first quality control primer, the second quality control primer, or both, comprises one or more polymerase stoppers. In some embodiments, each of the 3' overlapping regions of the first and second quality control primers comprises one or more polymerase stoppers. In some embodiments, at least one of the one or more polymerase stoppers is a 2'-O-methylated nucleotide.

The 3' overlapping region of the first quality control primer can be complementary to the 3' overlapping region of the second quality control primer. In some embodiments, the 3' overlapping region of the first quality control primer is fully complementary to the 3' overlapping region of the second quality control primer. In some embodiments, the 3' overlapping region of the first quality control primer and the 3' overlapping region of the second quality control primer have the same length. In some embodiments, the 3' overlapping region of the first quality control primer, the 3' overlapping region of the second quality control primer, or both, is about 2 to about 10 nucleotides in length, for example 4 or 5 nucleotides in length.

In some embodiments, (b) contacting the first quality control primer and the second quality control primer is carried out under the amplification condition. In some embodiments, (d) detecting the signal generated from the quenchable label of the first quality control primer during the amplification reaction comprises detecting the signal at two or more different time points during the amplification reaction. In some embodiments, the decrease in the signal during the amplification reaction comprises a decrease over time during the amplification reaction. In some embodiments, the decrease in the signal during the amplification reaction comprises a decrease over a time period of about 10 minutes during the amplification reaction. In some embodiments, the time period is between about 3 minutes to about 12 minutes from the start of the amplification reaction. The method can comprise detecting the signal of the quenchable label of the first quality control primer before the amplification reaction, after the amplification reaction, or both.

The amplification reaction can be, for example, a real-time amplification reaction, including a PCR reaction. In some embodiments, the amplification reaction comprises one or more of the following: archaeal polymerase amplification (APA), loop-mediated isothermal amplification (LAMP), helicase-dependent amplification (HDA), recombinase polymerase amplification (RPA), strand displacement amplification (SDA), nucleic acid sequence-based amplification (NASBA), transcription mediated amplification (TMA), nicking enzyme amplification reaction (NEAR), rolling circle amplification (RCA), multiple displacement amplification (MDA), Ramification (RAM), circular helicase-dependent amplification (cHDA), single primer isothermal amplification (SPIA), signal mediated amplification of RNA technology (SMART), self-sustained sequence replication (3SR), genome exponential amplification reaction (GEAR) and isothermal multiple displacement amplification (IMDA). The amplification reaction can be, for example, an isothermal amplification reaction. In some embodiments, the amplification condition comprises one or more of an enzyme having a hyperthermophile polymerase activity, dNTPs, and a buffering agent. In some embodiments, the enzyme having a hyperthermophile polymerase activity has an amino acid sequence that is at least about 90% or at least about 95% identical to the amino acid sequence of SEQ ID NO: 1 or a functional fragment thereof. In some embodiments, the enzyme having a hyperthermophile polymerase activity comprises the amino acid sequence of SEQ ID NO: 1.

The isothermal amplification reaction can comprise a constant temperature of about 30°C to about 72°C, including but not limited to, a constant temperature of about 67°C. The isothermal amplification reaction can be performed, for example, for a period of about 5 minutes, 6 minutes, 7 minutes, 8 minutes, 9 minutes, 10 minutes, 15 minutes, 20 minutes, 30 minutes, 40 minutes, 50 minutes, or 60 minutes. In some embodiments, the isothermal amplification reaction is performed in a helicase-free, single-stranded binding protein-free, cleavage agent-free, and recombinase-free, isothermal amplification condition.

In some embodiments, detecting the signal generated from the quenchable label of the first quality control primer does not comprise using any probe. In some embodiments, (c) subjecting the duplex to the amplification condition comprises subjecting a target nucleic acid and one or more additional primers and/or one or more probes specific to the target nucleic acid to the amplification condition. In some embodiments, the first quality control primer does not hybridize to the target nucleic acid under the amplification condition. In some embodiments, the second quality control primer does not hybridize to the target nucleic acid under the amplification condition. In some embodiments, the method does not comprise using any template nucleic acid capable of hybridizing to the first quality control primer. In some embodiments, the method does not comprise using any template nucleic acid capable of hybridizing to the second quality control primer.

Disclosed herein include kits for monitoring an amplification reaction. For example, the kit can comprise: a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label.

The quenchable label can be a fluorophore. In some embodiments, the quenchable label is outside of the 3' overlapping region of the first quality control primer. In some embodiments, the quenchable label is at 5' terminus of the first quality control primer. In some embodiments, the quenchable label is in the 3' overlapping region of the first quality control primer. In some embodiments, the second quality control primer comprises a quencher. In some embodiments, the quencher is outside of the 3' overlapping region of the second quality control primer. In some embodiments, the quencher is at 5' terminus of the second quality control primer. In some embodiments, the quencher is in the 3' overlapping region of the second quality control primer.

In some embodiments, the first quality control primer, the second quality control primer, or both, comprises one or more modified nucleotides. In some embodiments, each of the 3' overlapping regions of the first and second quality control primers comprises one or more modified nucleotides. In some embodiments, the one or more modified nucleotides comprise a spacer, an a-basic site, an un-methylated RNA base, a 2'-O-methylated nucleotide, and any combination thereof. In some embodiments, at least one of the one or more modified nucleotides is a 2'-O-methylated nucleotide. In some embodiments, the first quality control primer, the second quality control primer, or both, comprises one or more polymerase stoppers. In some embodiments, each of the 3' overlapping regions of the first and second quality control primers comprises one or more polymerase stoppers. In some embodiments, at least one of the one or more polymerase stoppers is a 2'-O-methylated nucleotide.

In some embodiments, the 3' overlapping region of the first quality control primer is complementary to the 3' overlapping region of the second quality control primer. In some embodiments, the 3' overlapping region of the first quality control primer is fully complementary to the 3' overlapping region of the second quality control primer. In some embodiments, the 3' overlapping region of the first quality control primer and the 3' overlapping region of the second quality control primer have the same length. In some embodiments, one or more of the 3' overlapping region of the first quality control primer and the 3' overlapping region of the second quality control primer is about 2 to about 10 nucleotides in length. In some embodiments, the 3' overlapping region of the first and second quality control primers is 4 or 5 nucleotides in length.

The kit can comprise one or more of an enzyme having a hyperthermophile polymerase activity, dNTPs, and a buffering agent. In some embodiments, the enzyme having a hyperthermophile polymerase activity has an amino acid sequence that is at least about 90% or at least about 95% identical to the amino acid sequence of SEQ ID NO: 1 or a functional fragment thereof. In some embodiments, the enzyme having a hyperthermophile polymerase activity comprises the amino acid sequence of SEQ ID NO: 1.

In some embodiments, the first quality control primer and the second quality control primer are in a lyophilized or freeze-dried form. In some embodiments, the one or more of an enzyme having a hyperthermophile polymerase activity, dNTPs, and a buffering agent are in a lyophilized or freeze-dried form. The kit can comprise one or more additional primers and/or one or more probes specific to a target nucleic acid.

Disclosed herein include reaction mixtures. In some embodiments, the reaction mixture comprises: a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label; a target nucleic acid; and one or more additional primers and/or one or more probes specific to the target nucleic acid.

The reaction mixture can comprise a duplex formed by hybridization between the 3' overlapping regions of the first and second quality control primers. The reaction mixture can comprise one or more of an enzyme having a hyperthermophile polymerase activity, dNTPs, and a buffering agent. In some embodiments, the enzyme having a hyperthermophile polymerase activity has an amino acid sequence that is at least about 90% or at least 95% identical to the amino acid sequence of SEQ ID NO: 1 or a functional fragment thereof. In some embodiments, the enzyme having a hyperthermophile polymerase activity comprises the amino acid sequence of SEQ ID NO: 1.

In some embodiments, the first quality control primer is not capable of hybridizing to the target nucleic acid. In some embodiments, the second quality control primer is not capable of hybridizing to the target nucleic acid. In some embodiments, the one or more additional primers and/or one or more probes are not capable of hybridizing to the first quality control primer, the second quality control primer, or both.

### BRIEF DESCRIPTION OF THE DRAWINGS

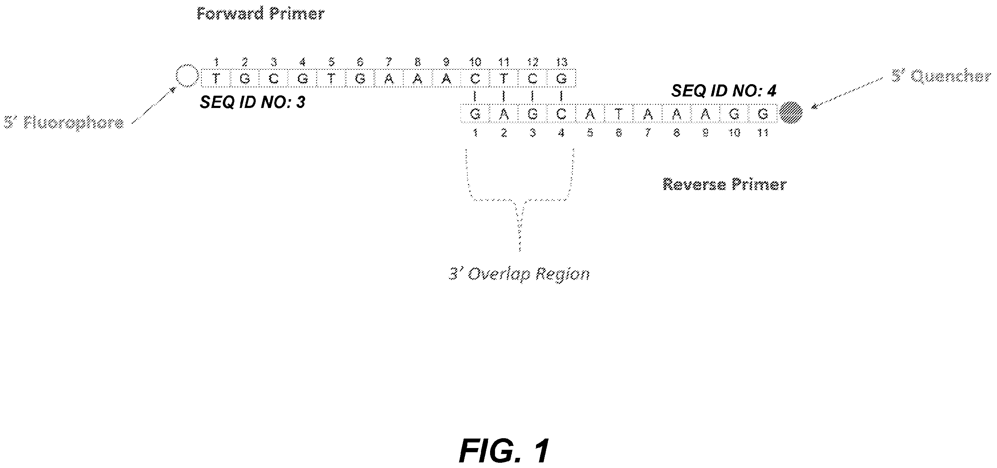

FIG. 1 is a non-limiting exemplary embodiment of a DIMER Internal Control (DIMER-IC) disclosed herein formed by a forward IC primer (associated with a fluorophore at the 5' terminus) and a reverse IC primer (associated with a quencher at the 5' terminus) capable of hybridizing to each other at the 3'-overlapping region to form a dimer.

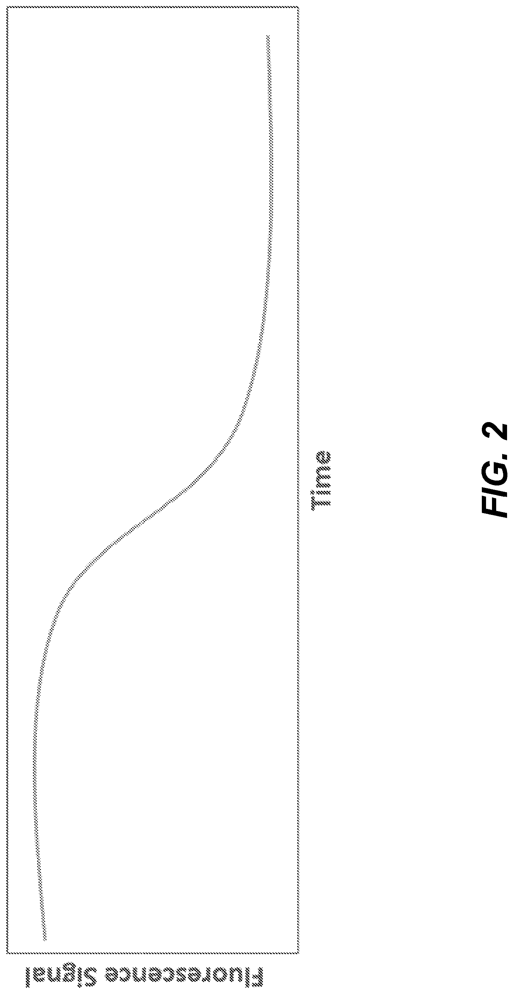

FIG. 2 depicts a non-limiting exemplary embodiment of the DIMER-IC method disclose herein, where fluorescence associated with the forward IC primer is quenched over time as the Archaeal Polymerase Amplification (APA) reaction proceeds to produce an extended duplex using the dimer formed by the forward and reverse IC primers as template.

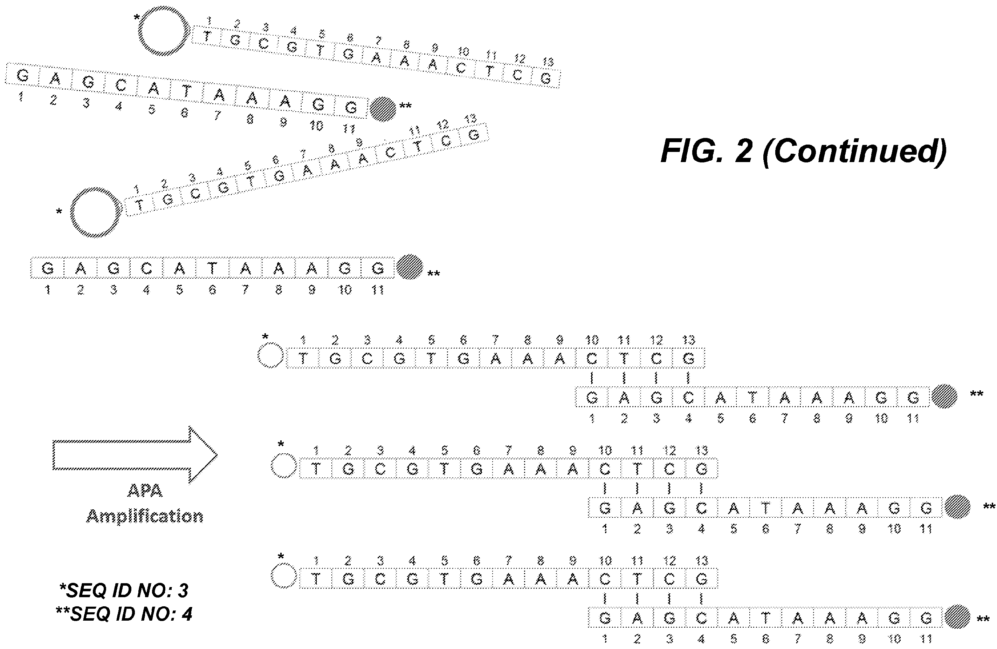

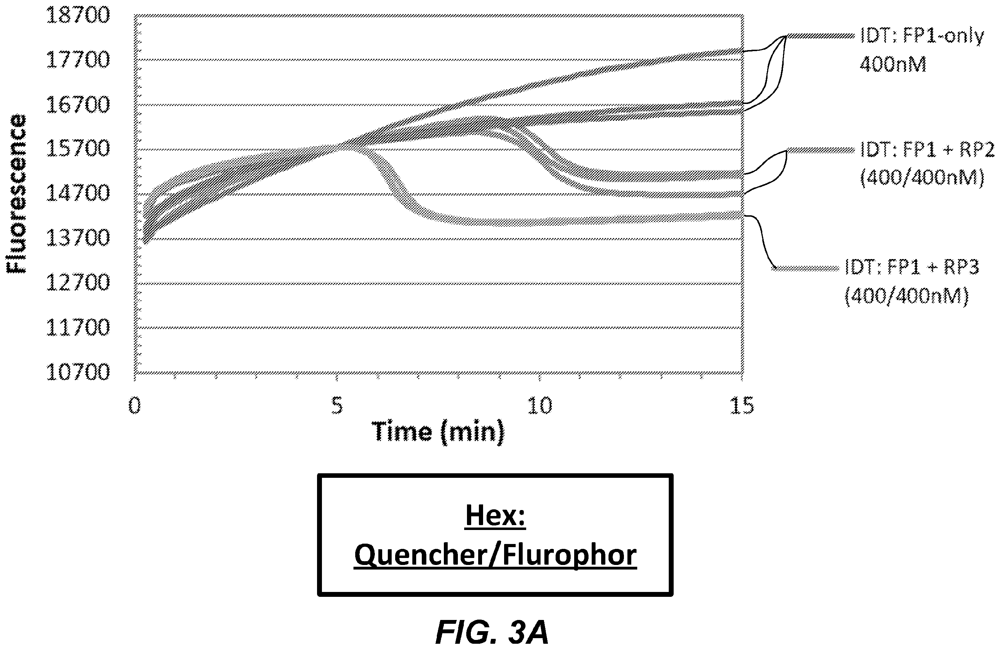

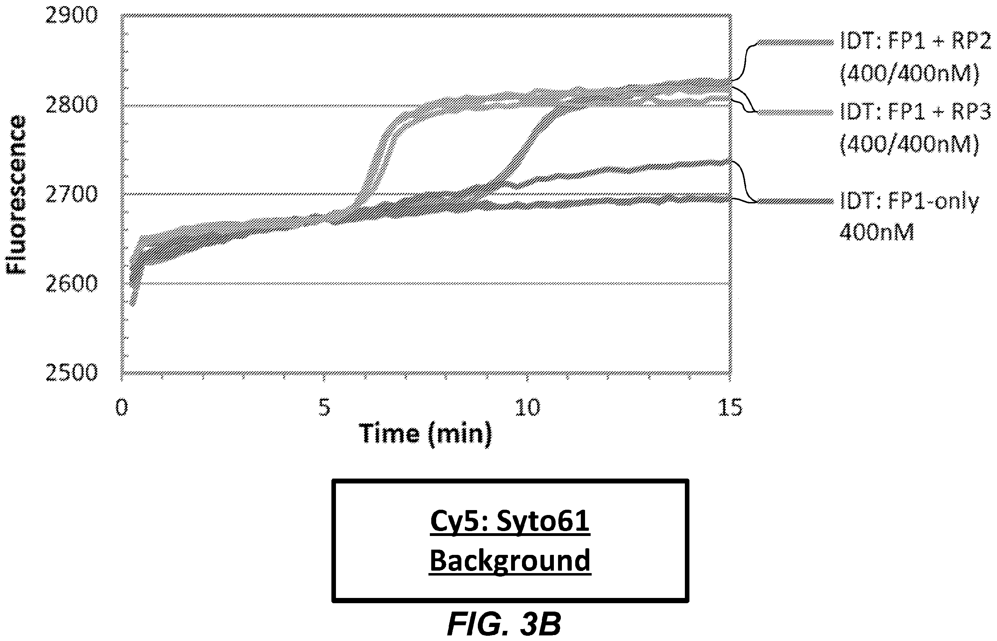

FIG. 3A-FIG. 3B depict inverted amplification curves produced by a DIMER-IC method described herein. FIG. 3A depicts the results using HEX fluorophore (FP1 + RP2 comprises a 4-bp 3' overlap. FP1 + RP3 comprise a 5-bp 3' overlap). FIG. 3B depicts the results using Syto61 intercalating dye (FP1 + RP2 comprises a 4-bp 3' overlap. FP1 + RP3 comprise a 5-bp 3' overlap).

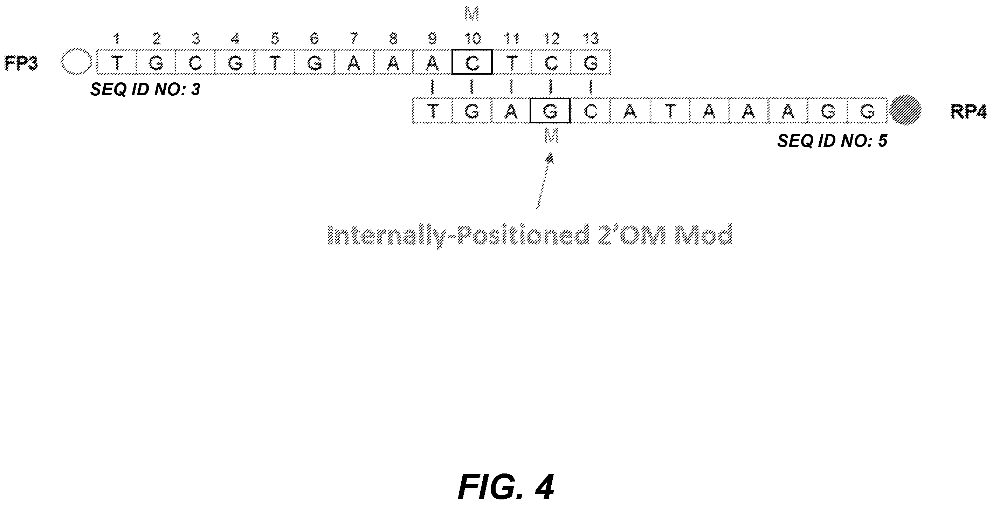

FIG. 4 depicts a non-limiting exemplary embodiment wherein one or more of the nucleotides in the 3'-overlapping regions of the forward and/or reverse IC primers comprises a modification (e.g., 2'-O-methylation).

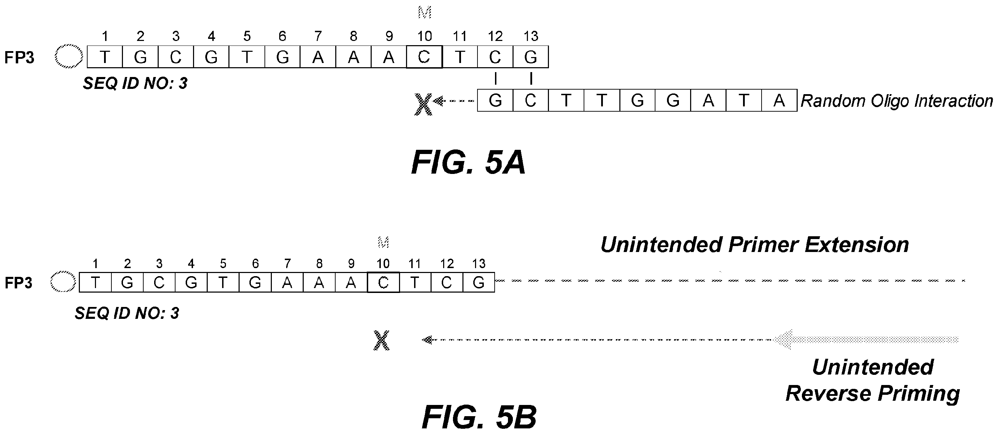

FIG. 5A-FIG. 5B show how modified nucleotides in the 3'-overlapping regions of the forward and/or reverse IC primers can inhibit unintended primer extension, in some embodiments.

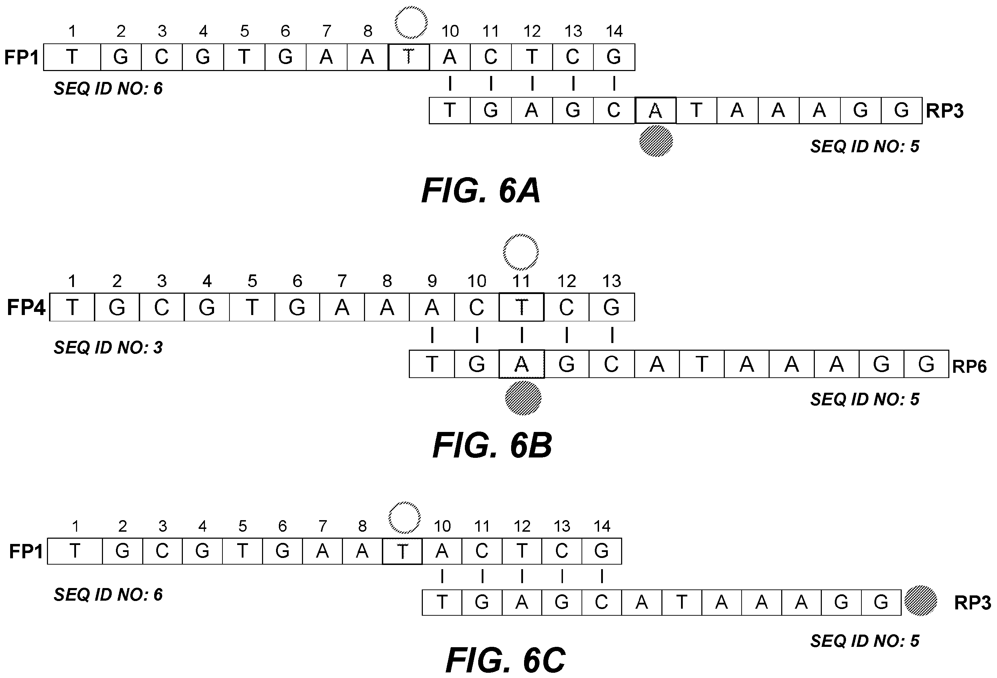

FIG. 6A-FIG. 6C depict non-limiting illustration showing alternative positions for the quenchable label and quencher associated with the forward and/or reverse IC primers: both outside the overlapping region (FIG. 6A), both within the overlapping regions (FIG. 6B), or one (e.g., the quencher) is at the 5' terminus of the reverse primer, and one (e.g., the fluorophore) is at an internal site (FIG. 6C).

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

Disclosed herein include methods, compositions, kits, and reactions mixtures for evaluating, monitoring, observing, and/or tracking the progress of nucleic acid amplification reactions.

Disclosed herein include methods for evaluating, monitoring, observing, and/or tracking the progress of an amplification reaction. In some embodiments, the method comprises: (a) providing a first quality control primer and a second quality control primer each comprising a 3' overlapping region capable of hybridizing to each other, wherein the first quality control primer comprises a quenchable label; (b) contacting the first quality control primer and the second quality control primer, thereby forming a duplex via hybridization between the 3' overlapping regions of the first and second quality control primers; (c) subjecting the duplex to an amplification condition, thereby generating an extended duplex; and (d) detecting a signal generated from the quenchable label of the first quality control primer during the amplification reaction to determine generation of the extended duplex, wherein a decrease in the signal during the amplification reaction indicates generation of the extended duplex. In some embodiments, each of the first quality control primer and the second quality control primer is no more than 35 nucleotides in length.

Disclosed herein include kits for evaluating, monitoring, observing, and/or tracking the progress of an amplification reaction. In some embodiments, the kit comprises: a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label.

Disclosed herein include reaction mixtures. In some embodiments, the reaction mixture comprises: a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label; a target nucleic acid; and one or more additional primers and/or one or more probes specific to the target nucleic acid.

The methods, compositions and kits described herein have various advantages over the currently available methods, compositions and kits for evaluating, monitoring, observing, and/or tracking the progress of nucleic acid amplification. The advantages include, but are not limited to: lower oligonucleotide complexity, no requirement for probe or target template, direct control of system via primer concentration and 3'-overlap size, possible for a universal IC system (i.e. higher degree of compatibility); less inhibitory effects (e.g., no Molecular Beacon probe(s) required, therefore less polymerase inhibition), and less susceptible to non-specific product formation (e.g., due to 2'-O-methyl (2'-OM) modification of primers).

### Definitions

Unless defined otherwise, technical and scientific terms used herein have the same meaning as commonly understood by one of ordinary skill in the art to which the present disclosure belongs. *See, e.g.* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "primer" refers to an oligonucleotide that includes a nucleotide sequence capable of hybridizing or annealing to a target nucleic acid, at or near (e.g., adjacent to) a specific region of interest.

As used herein, the term "quenchable label" refers to a molecule capable of producing a detectable signal, wherein the signal can be decreased by a quencher. As used herein, the term "quencher" can refer to molecules that interfere with or absorb the fluorescence emitted by a nearby quenchable label, e.g., a fluorophore.

As used herein, the term "duplex" refers to a region in two substantially complementary or fully complementary polynucleotides (e.g., 3' overlapping regions of a first quality control primer and a second quality control primer) that form base pairs with one another, either by Watson-Crick base pairing or any other manner that allows forming a stabilized duplex between polynucleotide strands that are substantially complementary or fully complementary. For example, a polynucleotide strand having 21 nucleotide units can base pair with another polynucleotide strand of 21 nucleotide units, when only 19 bases on each of the two strands can form Watson-Crick base pairing (i.e., fully complementary), such that the "duplex" has 19 base pairs. The remaining bases may, for example, exist as 5' and 3' overhangs. Further, within the duplex, full (e.g., 100%) complementarity is not required. For example, a mismatch in a duplex consisting of 19 base pairs results in 94.7% complementarity. In some embodiments, a duplex is formed via hybridization between the 3' overlapping regions of the first and second quality control primers. In some embodiments, the complementarity comprises 4-5 nucleotides in the 3' overlapping regions of the first and second quality control primers.

Provided herein include methods, compositions, kits, and reaction mixtures which can, in some embodiments, use Archaeal Polymerase Amplification ("APA") to isothermally amplify a region of interest within a target nucleotide template for the purpose of real-time analyte detection. For example, disclosed herein include methods for monitoring or evaluating an amplification reaction, e.g., as an Internal Control ("IC") assay, that takes advantage of a 3' overlap between quality control primers to initiate amplification (e.g., intentional primer-dimer formation), in conjunction with a probe-less, "integrated" detection format.

As disclosed herein, the quality control forward and reverse primers can, in some embodiments, comprise complementarity at the 3' ends (e.g., overlap of at least 4 (e.g., 4-6) base pairs), exponential amplification between these primers in the absence of any other target template can be consistently produced. In some embodiments, the speed and robustness of this amplification is correlated to the size of the 3' overlapping region (with a longer base-pair overlap correlated with increased amplification speed), as well as the overall concentration of these primers in solution (with higher concentration correlated to increased amplification speed).

APA amplicons are short, in some embodiments, no more than 35 base pairs (35bp) in total length. This is likewise true for the dimer-based internal control structure described above. To reduce assay complexity, the need for a dedicated control probe was advantageously eliminated in some embodiments described herein. For example, by tagging one of the primers with a 5' quenchable label (e.g., a fluorophore), and tagging the other primer with a 5' quencher, a signal response that was correlated with amplification (as indicated by inclusion of DNA intercalating dye within the same APA reaction) was produced. Without being bound by any particular theory, the generation of the DIME-IC control amplicon (e.g., an extended duplex) may bring the fluorophore and quencher into sufficient proximity to reduce fluorescence output (and thus an observable amplification response). Note that this yields an "inverse" exponential amplification curve, when compared to a standard intercalating curve response.

One of the advantages of the cumulative system for monitoring an amplification reaction using the method described herein is reduced assay complexity: a signal is generated from only two primers (no probe or dedicated templates are needed). This reduction in complexity can help to improve APA performance when the quality control primers and a target-analyte assay are co-amplifying in a common reaction vessel.

The methods, compositions, kits, and reaction mixtures for evaluating, monitoring, observing, and/or tracking an amplification reaction disclosed herein can, for example, be used to monitor amplification efficiency of a reaction. As used herein, the term "amplification efficiency" shall have its ordinary meaning, and also refers to a determination of the capacity of the amplification reaction to synthesize nucleic acids. Presence of amplification inhibitors, missing or defective amplification reaction components, nuclease contamination, and defective equipment comprise non-limiting examples of causes of reduced amplification efficiency, and can be detected by the methods, compositions, kits and reaction mixtures disclosed herein. In some embodiments, the methods, compositions, kits and reaction mixtures provided herein can be used for quantification purposes, e.g., production of the extended duplex can be used as quantification standard in real-time quantitative PCR or isothermal amplification applications.

### Quality control primers

Provided herein include quality control primers (also called "internal control primers" or "IC primers"). A pair of quality control primers, for example, can include a first quality control primer (e.g., a forward primer) and a second quality control primer (e.g., a reverse primer) each comprising a 3' overlapping region capable of hybridizing to each other. As described herein, the first quality control primer can be a forward primer or a reverse primer, and likewise the second quality control primer can be a forward primer or reverse primer. In a primer pair, if the first quality control primer is the forward primer, the second quality control primer is then the reverse primer, and vice versa. The length of the quality control primer can vary, for example, the quality control primer can be no more than 35 nucleotides in length. In some embodiments, the quality control primer (e.g., the forward primer) comprises a quenchable label. The methods, compositions, kits, and reaction mixtures described herein, in some embodiments, comprise providing a first quality control primer and a second quality control primer each comprising a 3' overlapping region capable of hybridizing to each other, wherein each of the first quality control primer and the second quality control primer is no more than 35 nucleotides in length, and wherein the first quality control primer comprises a quenchable label.

The quenchable label can be, for example, a fluorophore. As used herein, the term "fluorophore" shall be given its ordinary meaning and also refers to any reporter group whose presence can be detected by its light emitting properties. Non-limiting examples of fluorophore include: Cy2^{™} (506), YO-PRO^{™}.-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™} (531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™} (576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red(615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC (5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610) (the numbers in parentheses are maximum emission wavelength in nanometers for the responding fluorophore). In some embodiments, the fluorophore is Cy5^{™}. In some embodiments, the fluorophore is hexachlorofluorescein (HEX).

The location of the quenchable label in a quality control primer can vary in different embodiments. For example, the quenchable label can be outside of the 3' overlapping region of the quality control primer (e.g., the first quality control primer). In some embodiments, the quenchable label is at 5' terminus of the quality control primer. In some embodiments, the quenchable label is in the 3' overlapping region of the quality control primer.

The second quality control primer can comprise a quencher. Quenching can be mediated by fluorescence resonance energy transfer (FRET). FRET is based on classical dipole-dipole interactions between the transition dipoles of the donor (e.g., a fluorophore) and acceptor (e.g., a quencher) and is dependent on the donor-acceptor distance. FRET can typically occur over distances up to 100 Å. FRET also depends on the donor-acceptor spectral overlap and the relative orientation of the donor and acceptor transition dipole moments. Quenching of a fluorophore can also occur as a result of the formation of a non-fluorescent complex between a fluorophore and another fluorophore or non-fluorescent molecule. This mechanism is known as "contact quenching," "static quenching," or "ground-state complex formation." Without being bound by any particular theory, it is believed that a quencher moiety is not required in some embodiments of the method disclosed herein in order to observe a detectable change in fluorescence, and proximal-base quenching effects are sufficient to produce a detectable shift in fluorescence to allow evaluating, monitoring, observing, and/or tracking a nucleic acid amplification reaction. Quencher moieties are not required to elicit fluorescence quenching in some embodiments disclosed herein. For example, fluorescence quenching can be achieved by attaching the fluorophore to a cytosine nucleotide (e.g., a 5' cytosine) in a quality control primer (e.g., the first quality control primer), so that upon amplicon formation, a complimentary guanine nucleotide is incorporated directly opposite the fluorophore-labeled C base, thus quenching the fluorescent signal via "proximal G base" quenching.

Examples of the quencher include, but are not limited to, Iowa Black FQ, Iowa Black RQ, Black Hole Quencher-1 (BHQ-1), Black Hole Quencher-2 (BHQ-2), TMR, QSY-7, and Dabcyl. The location of the quencher in a quality control primer can vary. For example, the quencher can be outside of the 3' overlapping region of the quality control primer (e.g., the second quality control primer). In some embodiments, the quencher is at the 5' terminus of the quality control primer. In some embodiments, the quencher is in the 3' overlapping region of the quality control primer.

The length of the quality control primer (e.g., the first and second quality control primers) can vary, for example, from about 5 to about 100 nucleotides, including about 8, 10, 11, 12, 13, 14, 15, 16, 17, 18, 19, 20, 25, 30, 35, 40, 50, 75, 100 nucleotides, or a number or a range between any two of these values. In some embodiments, the first and second quality control primers each has a length of 10 to about 50 nucleotides, e.g., 10, 11, 12, 13, 14, 15, 16, 17, 18, 19, 20, 21, 22, 23, 24, 25 26, 27, 28, 29, 30, 31, 32, 33, 34, 35, 36, 37, 38, 39, 40, 41, 42, 43, 44, 45, 46, 47, 48, 49, or 50 nucleotides. In some embodiments, each of the first quality control primer and the second quality control primer is no more than 35 nucleotides in length.

### Modifications

The quality control primer can comprise or consist of modified nucleotide(s). The first quality control primer, the second quality control primer, or both, can comprise one or more modified nucleotides. For example, the 3' overlapping region of the first quality control primer, the 3' overlapping region of the second quality control primer, or both, can comprise one or more modified nucleotides. The location of the modification can be different in different embodiments. In some embodiments, each of the 3' overlapping regions of the first and second quality control primers comprises one or more modified nucleotides.

A nucleotide (or base) can be modified according to any modification described herein or known in the art. Modifications can include those made during primer synthesis and/or may include post-synthetic modifications. Modifications can include internal modifications, modifications at the 3' terminal region of a quality control primer, and/or modifications at the 5' terminal region of a quality control primer. In some embodiments, a quality control primer comprises a mixture of modified and unmodified nucleotides. In some embodiments, a quality control primer comprises unmodified nucleotides. In some embodiments, a quality control primer consists essentially of, or consists of, modified nucleotides.

Modifications and modified bases can include, for example, phosphorylation, (e.g., 3' phosphorylation, 5' phosphorylation); attachment chemistry or linkers modifications (e.g., Acrydite^{™}, adenylation, azide (NHS ester), digoxigenin (NHS ester), cholesteryl-TEG, I-Linker^{™}, amino modifiers (e.g., amino modifier C6, amino modifier C12, amino modifier C6 dT, Uni-Link^{™} amino modifier), alkynes (e.g., 5' hexynyl, 5-octadiynyl dU), biotinylation (e.g., biotin, biotin (azide), biotin dT, biotin-TEG, dual biotin, PC biotin, desthiobiotin-TEG), thiol modifications (e.g., thiol modifier C3 S-S, dithiol, thiol modifier C6 S-S)); spacers (C3 spacer, PC spacer, hexanediol, spacer 9, spacer 18, 1',2'-dideoxyribose (dSpacer); modified bases (e.g., 2-aminopurine, 2,6-diaminopurine (2-amino-dA), 5-bromo dU, deoxyUridine, inverted dT, inverted dideoxy-T, dideoxy-C, 5-methyl dC, deoxyInosine, Super T^{®}, Super G^{®}, locked nucleic acids (LNA's), 5-nitroindole, 2'-O-methyl RNA bases, hydroxmethyl dC, UNA unlocked nucleic acid (e.g., UNA-A, UNA-U, UNA-C, UNA-G), Iso-dC, Iso-dG, Fluoro C, Fluoro U, Fluoro A, Fluoro G); phosphorothioate bonds modifications (e.g., phosphorothioated DNA bases, phosphorothioated RNA bases, phosphorothioated 2' O-methyl bases, phosphorothioated LNA bases); and click chemistry modifications. In some embodiments, modifications and modified bases include uracil bases, ribonucleotide bases, O-methyl RNA bases, phosphorothioate linkages, 3' phosphate groups, spacer bases (e.g., C3 spacer or other spacer bases). The one or more modified nucleotides can comprise a spacer, an a-basic site, an un-methylated RNA base, a 2'-O-methylated nucleotide, and any combination thereof.

Primers can be directly synthesized that include 2'-O-methyl RNA bases. 2'-O-methyl RNA bases may be included in primers, for example, to inhibit read through by a DNA polymerase. In some embodiments, a quality control primer comprises one or more phosphorothioate (PS) linkages (e.g., phosphorothioate bond modifications). A PS bond substitutes a sulfur atom for a non-bridging oxygen in the phosphate backbone of a primer, which typically renders the internucleotide linkage resistant to nuclease degradation. Phosphorothioate bonds can be introduced between about the last 3 to 5 nucleotides at the 5'-end or the 3'-end of a quality control primer to inhibit exonuclease degradation, for example. Phosphorothioate bonds included throughout an entire primer can help reduce attack by endonucleases, in some embodiments. A quality control primer can, for example, comprise a 3' phosphate group. 3' phosphorylation can inhibit degradation by certain 3'-exonucleases and can be used to block extension by DNA polymerases, in certain instances. In some embodiments, a quality control primer comprises one or more spacer bases (e.g., one or more C3 spacers). A C3 spacer phosphoramidite can be incorporated internally or at the 5'-end of a primer. Multiple C3 spacers can be added at either end of a primer to introduce a long hydrophilic spacer arm for the attachment of fluorophores or other pendent groups, for example. In some embodiments, at least one of the one or more modified nucleotides is a C3 spacer, hexanediol, dSpacer, PC Spacer, Spacer 9, Spacer 18, 2-Aminopurine, 2, 6-Diaminopuyrine, dideoxycytidine, inverted dT, Iso-dG, Iso-dC, Inverted dideoxy-T, and 5-nitroindole. At least one of the one or more modified nucleotides can be a 2'-O-methylated nucleotide. In some embodiments, the 2'-O methylated nucleotide comprises one or more of 2'-O-methyluridine, 2'-O-methyladenosine, 2'-O-methylcytidine, and 2'-O-methylguanosine hydrate. As shown in FIG. 4-FIG. 5B, a nucleotide modification in the 3' overlapping region of the first and second quality control primers can, in some embodiments, inhibit unintended primer extension as a result of hybridization of an IC primer with a random oligonucleotide.

The quality control primer (e.g., the first quality control primer and/or the second quality control primer) can comprise one or more polymerase stoppers. Each of the 3' overlapping regions of the first and second quality control primers can comprise one or more polymerase stoppers. As used herein, a "polymerase stopper" is a molecule (e.g., a modified nucleotide) capable of terminating or inhibiting polymerization. In some embodiments, at least one of the one or more polymerase stoppers is a 2'-O-methylated nucleotide. Non-limiting examples of 2'-O methylated nucleotides include 2'-O-methyluridine, 2'-O-methyladenosine, 2'-O-methylcytidine, and 2'-O-methylguanosine.

### Quality control primer overlap, duplex, and extended duplex

In some embodiments, the methods, compositions, reaction mixtures, and kits provided herein comprise a first quality control primer and a second quality control primer each comprising a 3' overlapping region capable of hybridizing to each other. In some embodiments, the 3' overlapping region of the first quality control primer is complementary to the 3' overlapping region of the second quality control primer. The 3' overlapping region of the first quality control primer can be fully complementary to the 3' overlapping region of the second quality control primer.

Complementarity with respect to sequences generally refers to nucleotide sequences that will hybridize with each other. As used herein, the terms "anneal" or "hybridize" can refer to the formation of a stable complex between two molecules. The stringency of the hybridization conditions can be altered to tolerate varying amounts of sequence mismatch. In some embodiments, the 3' overlapping regions of the first and second quality control primers are at least 75% complementary to each other. For example, the 3' overlapping region of the first and second quality control primers can be, or be at least, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% complementary to each other.

Fully complementary generally refers to a nucleotide sequence in a first strand, for example, where each base in order (e.g., read 5' to 3') pairs with a correspondingly ordered base in a second strand, and there are no gaps, additional sequences or unpaired bases within the sequence considered as fully complementary. As used herein, fully complementary refers to all contiguous bases of a nucleotide sequence in a first stand (e.g., in the 3' overlapping region of the first quality control primer) being complementary to corresponding contiguous bases of a nucleotide sequence in a second strand (e.g., the 3' overlapping region of the second quality control primer). A fully complementary sequence can be about 5 to about 25 contiguous bases in length, including 5, 6, 7, 8, 9, 10, 11, 12, 13, 14, 15, 16, 17, 18, 19, 20, 21, 22, 23, 24, or 25 contiguous bases in length. In some embodiments, the 3' overlapping region of the first quality control primer and the 3' overlapping region of the second quality control primer have the same length. In some embodiments, the 3' overlapping region of the first quality control primer, the 3' overlapping region of the second quality control primer, or both, is 2 to about 10 nucleotides in length (e.g., about 2, 3, 4, 5, 6, 7, 8, 9, or 10 nucleotides). The 3' overlapping region of the first and second quality control primers can be, for example, 4 or 5 nucleotides in length.

In some embodiments, the method comprises one or more of contacting the first quality control primer and the second quality control primer, thereby forming a duplex via hybridization between the 3' overlapping regions of the first and second quality control primers; subjecting the duplex to an amplification condition, thereby generating an extended duplex; and contacting the first quality control primer and the second quality control primer is carried out under the amplification condition.

As described herein, the quality control primers can be used in the methods, compositions, kits and reaction mixtures disclosed herein for evaluating, monitoring, observing, and/or tracking nucleic acid amplification reactions. The nucleic acid amplification reactions can be, or comprise, one or more amplification reactions involving a target nucleic acid as template and one or more primers specific to the target nucleic acid. In some embodiments, the method disclosed herein (e.g., in the step of subjecting the duplex to the amplification condition) comprises subjecting a target nucleic acid and one or more additional primers and/or one or more probes specific to the target nucleic acid to the amplification condition. In some embodiments, it is advantageous that the first quality control primer, the second quality control primer, or both do not hybridize to the target nucleic acid under the amplification condition. In some embodiments, the method does not comprise using any nucleic acid (e.g., template nucleic acid) capable of hybridizing to the first quality control primer, the second quality control primer, or both. For example, the method, in some embodiments, does not comprise using any template nucleic acid that is substantially complementary or fully complementary to the first control primer, the second control primer, or both. It is advantageous in some embodiments that the first and second quality control primers are not complementary to the target nucleic acid, and are not capable of priming amplification reactions from a target nucleic acid sequence. In some embodiments, the 3' overlapping regions of the first and second quality control primers are not complementary to the target nucleic acid. In some embodiments, the 3' overlapping regions of the first and second quality control primers are not fully complementary to the target nucleic acid. For example, the first and second quality primer can comprise no more than 2, 3, 4, or 5 complementary nucleotides to the target nucleic acid sequence.

### Nucleic Acid Amplification

Disclosed herein include methods for evaluating, monitoring, observing, and/or tracking an amplification reaction. The method can comprise: (a) providing a first quality control primer and a second quality control primer each comprising a 3' overlapping region capable of hybridizing to each other, wherein the first quality control primer comprises a quenchable label; (b) contacting the first quality control primer and the second quality control primer, thereby forming a duplex via hybridization between the 3' overlapping regions of the first and second quality control primers. In some embodiments, the method comprises subjecting the duplex to an amplification condition, thereby generating an extended duplex. In some embodiments, each of the first quality control primer and the second quality control primer is no more than 35 nucleotides in length.

Disclosed herein include reaction mixtures of nucleic acid amplification. In some embodiments, the reaction mixture comprises: a first quality control primer and a second quality control primer each comprising a 3' overlapping region capable of hybridizing to each other, wherein the first quality control primer comprises a quenchable label; a target nucleic acid; and one or more additional primers and/or one or more probes specific to the target nucleic acid. As used herein, a primer or a probe specific to the target nucleic acid is a primer or a probe that is complementary or fully complementary to at least a portion of the target nucleic acid or the complement or reverse complement thereof. In some embodiments, each of the first quality control primer and the second quality control primer is no more than 35 nucleotides in length. The reaction mixture can comprise a duplex formed via hybridization between the 3' overlapping regions of the first and second quality control primers. The reaction mixture can comprise one or more of an enzyme having DNA polymerase activity (e.g., an enzyme having a hyperthermophile polymerase activity), dNTPs, and a buffering agent. In some embodiments, the reaction mixture comprises a reverse transcriptase. The enzyme having a hyperthermophile polymerase activity can have an amino acid sequence that is at least about 90% or at least 95% identical to the amino acid sequence of SEQ ID NO: 1 or a functional fragment thereof. In some embodiments, the enzyme having a hyperthermophile polymerase activity comprises, or consists of, the amino acid sequence of SEQ ID NO: 1. In some embodiments, the first quality control primer, the second quality control primer, or both, is not capable of hybridizing to the target nucleic acid. In some embodiments, the one or more additional primers and/or one or more probes are not capable of hybridizing to the first quality control primer, the second quality control primer, or both.

As used herein, nucleic acid amplification can refer to any known procedure for obtaining multiple copies of a target nucleic acid sequence or its complement or fragments thereof, using sequence-specific methods. Examples of nucleic acid amplification include, but are not limited to, polymerase chain reaction (PCR), ligase chain reaction (LCR), archaeal polymerase amplification (APA), loop-mediated isothermal amplification (LAMP), strand displacement amplification (SDA) (e.g., multiple displacement amplification (MDA)), replicase-mediated amplification, immuno-amplification, nucleic acid sequence based amplification (NASBA), self-sustained sequence replication (3SR), rolling circle amplification (RCA), helicase-dependent amplification (HDA), recombinase polymerase amplification (RPA), nicking enzyme amplification reaction (NEAR), Ramification (RAM), circular helicase-dependent amplification (cHDA), single primer isothermal amplification (SPIA), signal mediated amplification of RNA technology (SMART), genome exponential amplification reaction (GEAR) and isothermal multiple displacement amplification (IMDA), and transcription-mediated amplification (TMA). In some embodiments, two or more of the aforementioned nucleic acid amplification methods can be performed sequentially. The term "isothermal amplification reaction" shall be given its ordinary meaning and shall also include reactions wherein the temperature does not significantly change during the reaction. In some embodiments, the temperature of the isothermal amplification reaction does not deviate by more than 10°C, for example, by not more than 5°C and by not more than 2°C during the main enzymatic reaction step where amplification takes place. Depending on the method of isothermal amplification of nucleic acids, different enzymes can be used for amplification. Exemplary isothermal amplification compositions and methods are described in WO2017176404.

The amplification reaction can be a real-time amplification reaction, for example a real-time PCR reaction. PCR involves amplification of a target sequence (e.g., a target nucleic acid) using two or more extendable sequence-specific oligonucleotide primers that flank the target sequence. The nucleic acid containing the target sequence of interest is subjected to a program of multiple rounds of thermal cycling (denaturation, annealing and extension) in the presence of the primers, a thermostable DNA polymerase (e.g., Taq polymerase) and various dNTPs, resulting in amplification of the target sequence. PCR uses multiple rounds of primer extension reactions in which complementary strands of a defined region of a DNA molecule are simultaneously synthesized by a thermostable DNA polymerase. At the end of each cycle, each newly synthesized DNA molecule acts as a template for the next cycle. During repeated rounds of these reactions, the number of newly synthesized DNA strands increases exponentially such that after, for example, 20 to 30 reaction cycles, the initial template DNA will have been replicated several thousand-fold or million-fold. PCR can generate double-stranded amplification products suitable for post-amplification processing. Amplification products can be detected by, for example, visualization with agarose gel electrophoresis, by an enzyme immunoassay format using probe-based colorimetric detection, by fluorescence emission technology, or by other detection means known to one of skill in the art. Examples of PCR method include, but not limited to, Real-Time PCR, End-Point PCR, Amplified fragment length polymorphism PCR (AFLP-PCR), Alu-PCR, Asymmetric PCR, Colony PCR, DD-PCR, Degenerate PCR, Hot-start PCR, In situ PCR, Inverse PCR Long-PCR, Multiplex PCR, Nested PCR, PCR-ELISA, PCR-RFLP, PCR-single strand conformation polymorphism (PCR-SSCP), quantitative competitive PCR (QC-PCR), rapid amplification of cDNA ends-PCR (RACE-PCR), Random Amplification of Polymorphic DNA-PCR (RAPD-PCR), Real-Time PCR, Repetitive extragenic palindromic-PCR (Rep-PCR), reverse transcriptase PCR (RT-PCR), TAIL-PCR, Touchdown PCR and Vectorette PCR.

Real-time PCR, also called quantitative real time polymerase chain reaction (QRT-PCR), can be used to simultaneously quantify and amplify a specific part of a given nucleic acid molecule. It can be used to determine whether a specific sequence is present in the sample; and if it is present, the number of copies of the sequence that are present. The term "real-time" can refer to periodic monitoring during PCR. Certain systems such as the ABI 7700 and 7900HT Sequence Detection Systems (Applied Biosystems, Foster City, Calif.) conduct monitoring during each thermal cycle at a pre-determined or user-defined point. Real-time analysis of PCR with fluorescence resonance energy transfer (FRET) probes measures fluorescent dye signal changes from cycle-to-cycle. The real-time procedure follows the general pattern of PCR, but the nucleic acid is quantified after each round of amplification. Two examples of method of quantification are the use of fluorescent dyes (e.g., SYBR Green) that intercalate into dsDNA, and modified DNA oligonucleotide probes that fluoresce when hybridized with a complementary DNA. Intercalating agents have a relatively low fluorescence when unbound, and a relatively high fluorescence upon binding to double-stranded nucleic acids. As such, intercalating agents can be used to monitor the accumulation of double stranded nucleic acids during a nucleic acid amplification reaction. Examples of such non-specific dyes useful in the embodiments disclosed herein include intercalating agents such as SYBR Green I (Thermo Fisher), propidium iodide, ethidium bromide, and the like.

The terms "amplify", "amplification", "amplification reaction", or "amplifying" refer to any in vitro process for multiplying the copies of a target nucleic acid. Amplification sometimes refers to an exponential increase in target nucleic acid. However, "amplifying" may also refer to linear increases in the numbers of a target nucleic acid, but is different than a one-time, single primer extension step. In some embodiments a limited amplification reaction, also known as pre-amplification, can be performed. Pre-amplification is a method in which a limited amount of amplification occurs due to a small number of cycles, for example 10 cycles, being performed. Pre-amplification can allow some amplification, but stops amplification prior to the exponential phase, and typically produces about 500 copies of the desired nucleotide sequence(s). Use of pre-amplification can limit inaccuracies associated with depleted reactants in certain amplification reactions, and also can reduce amplification biases due to nucleotide sequence or species abundance of the target. In some embodiments a one-time primer extension is performed as a prelude to linear or exponential amplification.

In some embodiments of the methods described herein, primers and their corresponding target nucleic acid are contacted, and complementary sequences anneal or hybridize to one another. Primers can anneal to a target nucleic acid, at or near (e.g., adjacent to, abutting, and the like) a sequence of interest. A primer annealed to a target may be referred to as a primer-target hybrid, hybridized primer-target, or a primer-target duplex. The terms "near" or "adjacent to" when referring to a nucleotide sequence of interest refer to a distance (e.g., number of bases) or region between the end of the primer and the nucleotide or nucleotides (e.g., nucleotide sequence) of a target. Generally, adjacent is in the range of about 1 nucleotide to about 50 nucleotides, for example 1, 2, 3, 4, 5, 6, 7, 8, 9, 10, 15, 20, 25, 30, 35, 40, 45, 50, or a number or a range between any two of these values, nucleotides, away from a nucleotide or nucleotide sequence of interest. In some embodiments, primers in a set (e.g., a pair of primers, a forward and a reverse primer, a first oligonucleotide and a second oligonucleotide) anneal within about 1 to 20 nucleotides from a nucleotide or nucleotide sequence of interest and produce amplified products. In some embodiments, primers anneal within a nucleotide or a nucleotide sequence of interest. After annealing, each primer is extended along the target (i.e., template strand) by a polymerase to generate a complementary strand. Several cycles of primer annealing and extension can be carried out, for example, until a detectable amount of amplification product is generated. In some embodiments, where a target nucleic acid is RNA, a DNA copy (cDNA) of the target RNA is synthesized prior to or during the amplification step by reverse transcription. In some embodiments of the method described herein, quality control primers are contacted and hybridize to form a duplex. Each of the quality control primers can serve as the template for a nucleic acid extension reaction as well as the primer.

Components of an amplification reaction can include, for example, one or more primers (e.g., quality control primers, additional primers and/or probes comprising individual primers, primer pairs, primer sets, oligonucleotides, multiple primer sets for multiplex amplification, and the like), nucleic acid target(s) (e.g., target nucleic acid from a sample), one or more polymerases, nucleotides (e.g., dNTPs and the like), and a suitable buffer (e.g., a buffer comprising a detergent, a reducing agent, monovalent ions, and divalent ions). An amplification reaction can further include a reverse transcriptase and/or a reverse transcription primer. An amplification reaction can further include one or more detection agents, such as one or more of the detection agents described herein. In some embodiments, the one or more amplification reagents comprise, consist of, or consist essentially of primers, target nucleic acid, a polymerase, nucleotides, and a suitable buffer; and optionally a detection agent. In some embodiments, the one or more amplification reagents comprise, consist of, or consist essentially of, primers, target nucleic acid, a polymerase, a reverse transcriptase, a reverse transcription primer, nucleotides, and a suitable buffer; and optionally a detection agent and/or a reverse transcription primer. Additional components or features that do not have a significant effect on the amplification and/or are not necessary for generating a detectable product may be included. For example, additional components or features may be included that do not have a significant effect on the ability of the components and conditions herein to achieve amplification under isothermal conditions and generate a detectable amplification product within about 20, 15 or 10 minutes or less. Such additional components or features may be referred to as non-essential components and may include typical reaction components and/or common additives such as salts, buffers, detergents, ions, oils, proteins, polymers and the like.

Nucleic acid amplification can be conducted in the presence of native nucleotides, for example, dNTPs, and/or derivatized nucleotides. A native nucleotide generally refers to adenylic acid, guanylic acid, cytidylic acid, thymidylic acid, or uridylic acid. A derivatized nucleotide generally is a nucleotide other than a native nucleotide. Nucleotides typically are designated as follows. A ribonucleoside triphosphate is referred to as NTP or rNTP, where N can be A, G, C, U. A deoxynucleoside triphosphate substrates is referred to as dNTP, where N can be A, G, C, T, or U. Monomeric nucleotide subunits may be denoted as A, G, C, T, or U herein with no particular reference to DNA or RNA. In some embodiments, non-naturally occurring nucleotides or nucleotide analogs, such as analogs containing a detectable label (e.g., fluorescent or colorimetric label), may be used. For example, nucleic acid amplification may be carried out in the presence of labeled dNTPs, for example, radiolabels such as ³²P, ³³P, ¹²⁵I, or ³⁵S; enzyme labels such as alkaline phosphatase; fluorescent labels such as fluorescein isothiocyanate (FITC); or other labels such as biotin, avidin, digoxigenin, antigens, haptens, or fluorochromes. In some embodiments, nucleic acid amplification is carried out in the presence of modified dNTPs, for example, heat activated dNTPs (e.g., CleanAmp^{™} dNTPs from TriLink).

The one or more amplification reagents can include non-enzymatic components and enzymatic components. Non-enzymatic components can include, for example, primers, nucleotides, buffers, salts, reducing agents, detergents, and ions; and generally do not include proteins (e.g., nucleic acid binding proteins), enzymes, or proteins having enzymatic activity, for example, polymerases, reverse transcriptases, helicases, topoisomerases, ligases, exonucleases, endonucleases, restriction enzymes, nicking enzymes, recombinases and the like. Enzymatic components, in some embodiments, exclude other proteins (e.g., nucleic acid binding proteins and/or proteins having enzymatic activity), for example, helicases, topoisomerases, ligases, exonucleases, endonucleases, restriction enzymes, nicking enzymes, recombinases, and the like. Amplification conditions can comprise an enzymatic activity, for example an enzymatic activity provided by a polymerase. In some embodiments, an enzymatic activity is provided by a polymerase and a reverse transcriptase. In some embodiments, enzymatic activity does not include enzymatic activity provided by other enzymes, for example, helicases, topoisomerases, ligases, exonucleases, endonucleases, restriction enzymes, nicking enzymes, recombinases, and the like. The polymerase activity and a reverse transcriptase activity can be provided by separate enzymes or separate enzyme types (e.g., polymerase(s) and reverse transcriptase(s)); or by a single enzyme or enzyme type (e.g., polymerase(s)).

Traditional nucleic acid amplification methods typically require a thermocycling process, nucleic acid denaturation, proteins (e.g., enzymes) that promote strand unwinding, strand separation and/or strand exchange (e.g., helicases, recombinases), and/or endonuclease agents (e.g., restriction enzymes, nicking enzymes), and often require a minimum reaction time of 20 to 30 minutes. The nucleic acid amplification methods provided herein can be performed without thermocycling, without thermal denaturation and/or enzymatic denaturation of sample nucleic acids, without added proteins (e.g., enzymes) to promote strand unwinding, strand separation and/or strand exchange, without endonuclease agents, and/or within a reaction time of about 10-15 minutes. In some embodiments of the methods described herein, the amplification reaction is an isothermal amplification reaction. In some embodiments, amplification of nucleic acid comprises a non-thermocycling type of polymerase chain reaction (PCR). In some embodiments, amplification of nucleic acid comprises an isothermal amplification process. In some embodiments, amplification of nucleic acid comprises an isothermal polymerase chain reaction (iPCR).

Isothermal amplification conditions generally do not include a thermocycling (i.e., cycling between an upper temperature and a lower temperature) component in the amplification process, and therefore during an isothermal amplification reaction the temperature does not significantly change during the reaction (e.g., kept the temperature essentially the same during the reaction). For example, the temperature of the isothermal amplification reaction may not deviate by more than 10° C, for example by not more than 5° C or by not more than 2° C during the main enzymatic reaction step where amplification takes place. Depending on the method of isothermal amplification, different enzymes can be used for amplification. Nucleic acid amplification typically involves enzymatic synthesis of nucleic acid amplicons (copies), which contain a sequence complementary to a nucleotide sequence being amplified. The amplification method can be performed, for example, in a single vessel, a single chamber, and/or a single volume (i.e., contiguous volume). In some embodiments, the amplification and the detection (e.g., a detections step/method described herein) are performed (e.g., simultaneously) in a single vessel, a single chamber, and/or a single volume (i.e., contiguous volume). The amplification condition can comprise one or more of an enzyme having a hyperthermophile polymerase activity, dNTPs, and a buffering agent. The enzyme having a hyperthermophile polymerase activity can have an amino acid sequence that is at least about 90% or at least about 95% identical to the amino acid sequence of SEQ ID NO: 1 or a functional fragment thereof. The enzyme having a hyperthermophile polymerase activity can comprise the amino acid sequence of SEQ ID NO: 1.

The isothermal amplification reaction can be conducted at an essentially constant temperature. In some embodiments, the amplification reaction is maintained at precisely one temperature. In some embodiments, small fluctuations in temperature (e.g., ± 1 to 5°C) can occur in the isothermal amplification process due to, for example, environmental or equipment-based variables. It can be advantageous, in some embodiments, that the entire reaction volume is kept at an essentially constant temperature, and isothermal reactions herein generally do not include amplification conditions that rely on a temperature gradient generated within a reaction vessel and/or convective-flow based temperature cycling. In some embodiments, the isothermal amplification reaction is conducted at a temperature between about 30°C to about 75°C, including a temperature of about 55°C to a temperature of about 75°C. For example, the isothermal amplification reaction can be conducted at a temperature of, or of about, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, or 75°C, or a number between any two of these values. In some embodiments, the isothermal amplification reaction is conducted at a temperature of about 55°C to a temperature of about 65°C, for example at a temperature of about 60°C or about 65°C. In some embodiments, a temperature element (e.g., heat source) is kept at an essentially constant temperature, for example at an essentially constant temperature at or below about 75°C, for example at or below about 70°C, at or below about 65°C, or at or below about 60°C. The isothermal amplification reaction can be performed for a period of about 5, 6, 7, 8, 9, 10, 11, 12, 13, 14, 15, 16, 17, 18, 19, 20, 25, 30, 35, 40, 45, 50, 55, or 60 minutes, or a number or a range between any two of these values. The isothermal amplification reaction can be performed in a helicase-free, single-stranded binding protein-free, cleavage agent-free, and/or recombinase-free, isothermal amplification condition.

An amplification process described herein (e.g., an isothermal amplification) can be conducted over a suitable duration of time. In some embodiments, an amplification process is conducted until a detectable nucleic acid amplification product is generated. A nucleic acid amplification product may be detected by any suitable detection process and/or a detection process described herein. In some embodiments, an amplification process is conducted over a length of time within about 30 minutes, about 20 minutes or less. For example, an amplification process can be conducted within, or within about, 1, 2, 3, 4, 5, 6, 7, 8, 9, 10, 11, 12, 13, 14, 15, 16, 17, 18, 19, or 20 minutes. In some embodiments, the amplification generates detectable nucleic acid amplification products within about 20, 19, 18, 17, 16, 15, 14, 13, 12, 11, 10, 9, 8, 7, 6, or 5 minutes, or less, or a number or a range between any two of these values.

Nucleic acid targets and duplexes (e.g., duplexes formed by hybridization between the 3' overlapping regions of the first and second quality control primers) can be amplified without exposure to agents or conditions that denature nucleic acid, in some embodiments. Nucleic acid targets and duplexes can be amplified without exposure to agents or conditions that promote strand separation during the amplification step (and/or other steps) in some embodiments. Nucleic acid targets and duplexes can be amplified without exposure to agents or conditions that promote unwinding during the amplification step (and/or other steps) in some embodiments. Agents or conditions that denature nucleic acid and/or promote strand separation and/or promote unwinding include, but are not limited to, thermal conditions (e.g., high temperatures), pH conditions (e.g., high or low pH), chemical agents, and proteins (e.g., enzymatic agents).

In some embodiments, the method disclosed herein (e.g., the amplifying step) do not comprise thermal denaturation (e.g., heating a solution containing a nucleic acid to an elevated temperature, for example a temperature above 75°C, above 80°C, above 90°C, above 95°C, or higher) or protein-based (e.g., enzymatic) denaturation of a nucleic acid. Protein-based (e.g., enzymatic) denaturation can comprise contacting a nucleic acid with one or more of a helicase, a topoisomerase, a ligase, an exonuclease, an endonuclease, a restriction enzyme, a nicking enzyme, a recombinase, a RNA replicase, and a nucleic acid binding protein (e.g., single-stranded binding protein). In some embodiments, the method does not comprise a helicase, a topoisomerase, a ligase, an exonuclease, an endonuclease, a restriction enzyme, a nicking enzyme, a recombinase, a RNA replicase, and/or a nucleic acid binding protein (e.g., single-stranded binding protein). In some embodiments, the method do not comprise intercalators, alkylating agents, and/or chemicals such as formamide, glycerol, urea, dimethyl sulfoxide (DMSO), and/or N,N,N-trimethylglycine (betaine). In some embodiments, the method does not comprise contacting a nucleic acid with denaturing agents (e.g., formamide). In some embodiments, the method (e.g., the amplifying step) and composition disclosed herein do not comprise agents and/or conditions that denature nucleic acids (e.g., promote strand separation and/or promote unwinding), or agents and/or conditions other than acids and/or low pH conditions. In some embodiments, the amplifying step does not comprise agents and/or conditions that denature nucleic acids (e.g., promote strand separation and/or promote unwinding) other than a polymerase (e.g., a hyperthermophile polymerase). In some embodiments, the methods and compositions provided herein not comprise agents and/or conditions that denature nucleic acids (e.g., promote strand separation and/or promote unwinding) other than a polymerase (e.g., a hyperthermophile polymerase) and/or low pH conditions (e.g., contact with acid(s)). Compositions, kits, and methods for nucleic acid detection wherein nucleic acid strands are dissociated under low pH conditions (e.g., via contact with an acidic lysis buffer) to facilitate subsequent rapid amplification and detection are described in PCT Patent Application Number PCT/US23/61978, entitled "METHOD FOR SEPARATING GENOMIC DNA FOR AMPLIFICATION OF SHORT NUCLEIC ACID TARGETS" and filed on February 3, 2023.

Nucleic acid targets and duplexes (e.g., duplexes formed by hybridization between the 3' overlapping regions of the first and second quality control primers) can be amplified without exposure to agents or conditions during the amplification step (and/or other steps) that promote strand separation and/or unwinding, in some embodiments. For example, nucleic acids can be amplified without exposure to a helicase. Helicases are enzymes capable of unwinding and separating double-stranded nucleic acid into single strands. Amplification conditions that do not include use of a helicase may be referred to herein as helicase-free amplification conditions.

Nucleic acid targets (including duplexes formed by hybridization between the 3' overlapping regions of the first and second quality control primers) can be amplified without being contacted with, or exposed to, one or more of helicase, recombinase, nucleic acid binding protein (e.g., single-stranded binding protein or single-strand DNA-binding protein (SSB), ligase, RNA replicase, restriction enzyme, nicking enzyme, exonuclease, DNAse, RNAse, and topoisomerase, prior to, and/or during the amplification reaction. Non-limiting examples of recombinases include Cre recombinase, Hin recombinase, Tre recombinase, FLP recombinase, RecA, RAD51, RadA, T4 uvsX. In some embodiments, nucleic acids may be amplified without exposure to a recombinase accessory protein, for example, a recombinase loading factor (e.g., T4 uvsY). In some embodiments, nucleic acids are amplified without exposure to a SSB, for example, T4 gp32. Non-limiting examples of exonucleases include DNAses, RNAses (e.g., RNAseH), 5' to 3' exonucleases (e.g. exonuclease II), 3' to 5' exonucleases (e.g. exonuclease I), and poly(A)-specific 3' to 5' exonucleases.

Nucleic acids targets (including duplexes formed by hybridization between the 3' overlapping regions of the first and second quality control primers) can be amplified with or without exposure to agents or conditions that destabilize nucleic acid. As used herein, the term "destabilization" shall be given its ordinary meaning, and also refer to a disruption in the overall organization and geometric orientation of a nucleic acid molecule (e.g., double helical structure) by one or more of tilt, roll, twist, slip, and flip effects. Destabilization generally does not refer to melting or separation of nucleic acid strands (e.g., denaturation). Nucleic acid destabilization can be achieved, for example, by exposure to agents such as intercalators or alkylating agents, and/or chemicals such as formamide, urea, dimethyl sulfoxide (DMSO), or N,N,N-trimethylglycine (betaine). In some embodiments, methods provided herein include use of one or more destabilizing agents. In some embodiments, methods provided herein exclude use of destabilizing agents.

Nucleic acid targets and duplexes (e.g., duplexes formed by hybridization between the 3' overlapping region of the first and second quality control primers) can be amplified as a template nucleic acid without cleavage or digestion, in some embodiments. For example, amplification can occur without prior exposure to one or more cleavage agents, and intact nucleic acid is amplified. In some embodiments, amplification can occur without exposure to one or more cleavage agents during amplification. The term "cleavage agent" refers to an agent (e.g., a chemical or an enzyme) that can cleave a nucleic acid at one or more specific or non-specific sites. Specific cleavage agents often cleave specifically according to a particular nucleotide sequence at a particular site. Cleavage agents include, but are not limited to, endonucleases (e.g., restriction enzymes, nicking enzymes, and the like); exonucleases (DNAses, RNAses (e.g., RNAseH), 5' to 3' exonucleases (e.g. exonuclease II), 3' to 5' exonucleases (e.g. exonuclease I), and poly(A)-specific 3' to 5' exonucleases); and chemical cleavage agents.

An amplified nucleic acid (e.g., an amplified target nucleic acid and/or an extended duplex) may be referred to herein as a nucleic acid amplification product or amplicon. An amplified duplex may be referred to as an extended duplex. The amplification product can include naturally occurring nucleotides, non-naturally occurring nucleotides, nucleotide analogs, and a combination thereof. An amplification product typically has a nucleotide sequence that is identical to or substantially identical to a sequence in a sample nucleic acid (e.g., target nucleic acid) or complement thereof. A "substantially identical" nucleotide sequence in an amplification product will generally have a high degree of sequence identity to the nucleotide sequence being amplified or complement thereof (e.g., about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or greater than 99% sequence identity), and variations sometimes are a result of polymerase infidelity or other variables.

In some embodiments, a nucleic acid amplification product comprises a polynucleotide that is complementary or fully complementary to a target sequence in sample nucleic acid. In some embodiments, a nucleic acid amplification product comprises a polynucleotide that is partially complementary or fully complementary to a duplex. Fully complementary refers to a nucleotide sequence in a first strand, for example, where each base in order (e.g., read 5' to 3') pairs with a correspondingly ordered base in a second strand, and there are no gaps, additional sequences or unpaired bases within the sequence considered as fully complementary. Stated another way, fully complementary refers to all contiguous bases of a nucleotide sequence in a first stand being complementary to corresponding contiguous bases of a nucleotide sequence in a second strand. Nucleic acid amplification products can comprise sequences fully complementary to or complementary to one or more primers used in an amplification reaction. In some embodiments, a nucleic acid amplification product comprises a first nucleotide sequence that is fully complementary to or identical to a first primer sequence, and a second nucleotide sequence that is fully complementary to or identical to a second primer sequence.

Nucleic acid amplification products can be at least 50 bases in length. In some embodiments, a nucleic acid amplification product is about 15 to about 40 bases long, for example, be, or be about, 15, 16, 17, 18, 19, 20, 21, 22, 23, 24, 25, 26, 27, 28, 29, 30, 31, 32, 33, 34, 35, 36, 37, 38, 39, or 40 bases long. In some embodiments, an amplification product is about 20 to about 40 bases long, for example about 20 to about 30 bases long. In some embodiments, nucleic acid amplification products for a given target sequence have the same length or substantially the same length (e.g., within 1 to 5 bases). Accordingly, nucleic acid amplification products for a given target sequence may produce a single signal (e.g., band on an electrophoresis gel) and generally do not produce multiple signals indicative of multiple lengths (e.g., a ladder or smear on an electrophoresis gel). For multiplex reactions, nucleic acid amplification products for different target sequences may have different lengths.

Nucleic acid amplification products can comprise a spacer sequence. As described herein, a spacer sequence in an amplification product is a sequence (1 or more bases) continuously complementary to or substantially identical to a portion of a target sequence in the sample nucleic acid, and is flanked by sequences in the amplification product that are complementary to or substantially identical to one or more primers used in an amplification reaction. A spacer sequence flanked by sequences in the amplification product generally lies between a first sequence (complementary to or substantially identical to a first primer) and a second sequence (complementary to or substantially identical to a second primer). Thus, an amplification product typically includes a first sequence followed by a spacer sequences followed by a second sequence. A spacer sequence generally is not complementary to or substantially identical to a sequence in the primer(s). A spacer sequence can be, or can comprise, about 1 to 10 bases, including 1, 2, 3, 4, 5, 6, 7, 8, 9, or 10 bases. In some embodiments, a nucleic acid amplification product consists of, or consists essentially of, a first nucleotide sequence that is continuously complementary to or identical to a first primer sequence, a second nucleotide sequence that is continuously complementary to or identical to a second primer sequence, and a spacer sequence. In some embodiments, a nucleic acid amplification product does not include any additional sequences (e.g., at the 5' and/or 3' end; or within the product) that are not continuously complementary to or identical to a first primer sequence and a second primer sequence, and are not part of a spacer sequence, for example, additional sequences incorporated into an amplification product by way of tailed or looped primers, ligation or other mechanism. In some embodiments, a nucleic acid amplification product generally does not include additional sequences (e.g., at the 5' and/or 3' end; or within the product) that are not continuously complementary to or identical to a first primer sequence and a second primer sequence, and are not part of a spacer sequence, for example, additional sequences incorporated into an amplification product by way of tailed or looped primers, ligation or other mechanism. However, in such embodiments, a nucleic acid amplification product may include, for example, some mismatched (i.e., non-complementary) bases or one more extra bases (e.g., at the 5' and/or 3' end; or within the product) introduced into the product by way of error or promiscuity in the amplification process.

The methods and components described herein can be used for evaluating and/or monitoring multiplex amplification in which the amplification of more than one nucleic acid of interest (e.g., amplification of more than one target sequence) occurs. For example, multiplex amplification can refer to amplification of multiple sequences from the same sample or amplification of one of several sequences in a sample. Multiplex amplification also may refer to amplification of one or more sequences present in multiple samples either simultaneously or in step-wise fashion. In some instances, an amplification reaction may be prepared to detect at least two target sequences, but only one of the target sequences may be present in the sample being tested, such that both sequences are capable of being amplified, but only one sequence is amplified. In some instances, where two target sequences are present, an amplification reaction may result in the amplification of both target sequences. A multiplex amplification reaction may result in the amplification of one, some, or all of the target sequences for which it comprises the appropriate primers and enzymes. In some instances, an amplification reaction may be prepared to detect two sequences with one pair of primers, where one sequence is a target sequence and one sequence is a control sequence (e.g., a synthetic sequence capable of being amplified by the same primers as the target sequence and having a different spacer base or sequence than the target). In some embodiments, the control sequence comprises the duplex formed by hybridization of the first and second quality control primers. In some instances, an amplification reaction may be prepared to detect multiple sets of sequences with corresponding primer pairs, where each set includes a target sequence and a control sequence.

### Primers

In some embodiments of the methods described herein, subjecting the duplex to the amplification condition can comprise subjecting a target nucleic acid and one or more additional primers and/or one or more probes specific to the target nucleic acid to the amplification condition.

Nucleic acid amplification generally is conducted in the presence of one or more primers. A primer (e.g., one or more additional primers) is generally characterized as an oligonucleotide that includes a nucleotide sequence capable of hybridizing or annealing to a target nucleic acid, at or near (e.g., adjacent to) a specific region of interest (i.e., target nucleic acid). Primers can allow for specific determination of a target nucleic acid nucleotide sequence or detection of the target nucleic acid (e.g., presence or absence of a sequence), or feature thereof, for example. A primer may be naturally occurring or synthetic. The term specific, or specificity, generally refers to the binding or hybridization of one molecule to another molecule, such as a primer for a target polynucleotide. That is, specific or specificity refers to the recognition, contact, and formation of a stable complex between two molecules, as compared to substantially less recognition, contact, or complex formation of either of those two molecules with other molecules. The term anneal or hybridize generally refers to the formation of a stable complex between two molecules. The terms primer, oligo, or oligonucleotide may be used interchangeably herein, when referring to primers.

A primer may be designed and synthesized using suitable processes, and may be of any length suitable for hybridizing to a target sequence and performing an amplification process described herein. Primers often are designed according to a sequence in a target nucleic acid. A primer in some embodiments can be about 5 bases in length to about 30 bases in length, for example 5, 6, 7, 8, 9, 10, 11, 12, 13, 14, 15, 16, 17, 18, 19, 20, 21, 22, 23, 24, 25, 26, 27, 28, 29 or 30 bases in length. In some embodiments, a primer is less than 28 bases in length, for example, about 8 to about 16 bases or about 10 to about 12 bases in length. A primer can be composed of naturally occurring and/or non-naturally occurring nucleotides (e.g., modified nucleotides, labeled nucleotides), or a mixture thereof. Primers suitable for use with methods described herein may be synthesized and labeled using any suitable technique. For example, a primer can be chemically synthesized according to the solid phase phosphoramidite triester method, using an automated synthesizer. Purification of primers can be effected, for example, by native acrylamide gel electrophoresis or by anion-exchange HPLC.

A primer can comprise or consist of modified nucleotides. A nucleotide (or base) may be modified according to any modification described herein or known in the art. Modifications can include those made during primer synthesis and/or can include post-synthetic modifications. Modifications can include internal modifications, modifications at the 3' end of a primer, and/or modifications at the 5' end of a primer. In some embodiments, a primer comprises a mixture of modified and unmodified nucleotides. In some embodiments, a primer comprises unmodified nucleotides. Modifications and modified bases can include, for example, phosphorylation, (e.g., 3' phosphorylation, 5' phosphorylation); attachment chemistry or linkers modifications (e.g., Acrydite^{™}, adenylation, azide (NHS ester), digoxigenin (NHS ester), cholesteryl-TEG, I-Linker^{™}, amino modifiers (e.g., amino modifier C6, amino modifier C12, amino modifier C6 dT, Uni-Link^{™} amino modifier), alkynes (e.g., 5' hexynyl, 5-octadiynyl dU), biotinylation (e.g., biotin, biotin (azide), biotin dT, biotin-TEG, dual biotin, PC biotin, desthiobiotin-TEG), thiol modifications (e.g., thiol modifier C3 S-S, dithiol, thiol modifier C6 S-S)); fluorophores (e.g., Freedom^{™} Dyes, Alexa Fluor^{®} Dyes, LI-COR IRDyes^{®}, ATTO^{™} Dyes, Rhodamine Dyes, WellRED Dyes, 6-FAM (azide), Texas Red^{®}-X (NHS ester), Lightcycler^{®} 640 (NHS ester), Dy 750 (NHS ester)); Iowa Black^{®} dark quenchers modifications (e.g., Iowa Black^{®} FQ, Iowa Black^{®} RQ); dark quenchers modifications (e.g., Black Hole Quencher^{®}-1, Black Hole Quencher^{®}-2, Dabcyl); spacers (C3 spacer, PC spacer, hexanediol, spacer 9, spacer 18, 1',2'-dideoxyribose (dSpacer); modified bases (e.g., 2-aminopurine, 2,6-diaminopurine (2-amino-dA), 5-bromo dU, deoxyUridine, inverted dT, inverted dideoxy-T, dideoxy-C, 5-methyl dC, deoxyInosine, Super T^{®}, Super G^{®}, locked nucleic acids (LNA's), 5-nitroindole, 2'-O-methyl RNA bases, hydroxmethyl dC, UNA unlocked nucleic acid (e.g., UNA-A, UNA-U, UNA-C, UNA-G), Iso-dC, Iso-dG, Fluoro C, Fluoro U, Fluoro A, Fluoro G); phosphorothioate bonds modifications (e.g., phosphorothioated DNA bases, phosphorothioated RNA bases, phosphorothioated 2' O-methyl bases, phosphorothioated LNA bases); and click chemistry modifications. In some embodiments, modifications and modified bases include uracil bases, ribonucleotide bases, O-methyl RNA bases, phosphorothioate linkages, 3' phosphate groups, spacer bases (such as C3 spacer or other spacer bases). For example, a primer cn comprise one or more O-methyl RNA bases (e.g., 2'-O-methyl RNA bases). 2'-O-methyl RNA generally is a post-transcriptional modification of RNA found in tRNA and other small RNAs. Primers can be directly synthesized that include 2'-O-methyl RNA bases. This modification can, for example, increase Tm of RNA:RNA duplexes and provide stability in the presence of single-stranded ribonucleases and Dnases. 2'-O-methyl RNA bases may be included in primers, for example, to increase stability and binding affinity to a target sequence. In some embodiments, a primer may comprise one or more phosphorothioate linkages (e.g., phosphorothioate bond modifications). A phosphorothioate (PS) bond substitutes a sulfur atom for a non-bridging oxygen in the phosphate backbone of a primer. This modification typically renders the internucleotide linkage resistant to nuclease degradation. Phosphorothioate bonds may be introduced between about the last 3 to 5 nucleotides at the 5'-end or the 3'-end of a primer to inhibit exonuclease degradation, for example. Phosphorothioate bonds included throughout an entire primer can help reduce attack by endonucleases, in some embodiments. A primer can, for example, comprise a 3' phosphate group. 3' phosphorylation can inhibit degradation by certain 3'-exonucleases and can be used to block extension by DNA polymerases, in certain instances. In some embodiments, a primer comprises one or more spacer bases (e.g., one or more C3 spacers). A C3 spacer phosphoramidite can be incorporated internally or at the 5'-end of a primer. Multiple C3 spacers can be added at either end of a primer to introduce a long hydrophilic spacer arm for the attachment of fluorophores or other pendent groups, for example.

A primer (e.g., a quality control primer or a target sequence-specific primer) can comprise DNA bases, RNA bases, or both. In some embodiments, a primer comprises a mixture of DNA bases and RNA bases. The DNA base and/or the RNA base can be modified or unmodified. In some embodiments, a primer consist of DNA bases (e.g., modified DNA bases and/or unmodified DNA bases). In some embodiments, a primer consists of RNA bases (e.g., modified RNA bases and/or unmodified RNA bases). In some embodiments, a primer comprises no RNA bases. In some embodiments, a primer comprises no RNA bases at the 3' end. In some embodiments, a primer comprises a DNA base (or modified DNA base) at the 3' end. In some embodiments, a primer is not a chimeric primer. A chimeric primer is a primer comprising DNA and RNA bases. In some embodiments, a primer is a homogeneous primer (e.g., a homogeneous DNA primer). A homogeneous DNA primer can comprise unmodified DNA bases, modified DNA bases, or a mixture of modified DNA bases and unmodified DNA bases, and do not include RNA bases.

In some embodiments, a primer comprises no cleavage agent recognition sites. For example, a primer can comprise no nicking enzyme recognition sites. In some embodiments, a primer comprises no tail. In some embodiments, a primer comprises no tail comprising a nicking enzyme recognition site.

All or a portion of a primer sequence may be complementary or fully complementary to a target nucleic acid, in some embodiments. Complementarity with respect to sequences generally refers to nucleotide sequences that will hybridize with each other. The stringency of the hybridization conditions can be altered to tolerate varying amounts of sequence mismatch. In some embodiments, target and primer sequences are at least 75% complementary to each other. For example, target and primer sequences may be, or be at least, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% complementary to each other. Primers that are complimentary to a target nucleic acid sequence typically are also complementary or fully complementary to the compliment of the target nucleic acid sequence (e.g., the anti-sense strand). Primers that are complementary to the anti-sense strand of a target nucleic acid may be, be about, be at least, or be at least about, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to each other. Whether two nucleotide sequences are complementary can be determined by the percent of identical nucleotide sequences shared.

As described herein, a pair of primers can include a forward primer and a reverse primer (e.g., primers that bind to the sense and antisense strands of a target nucleic acid). Because of the complementarity at the 3' overlapping region, a forward quality control primer can bind to a corresponding reverse quality primer, and the forward and reverse quality primers are a pair of quality control primers. In some embodiments, primers consist of a pair of primers (i.e. a forward primer and a reverse primer). Accordingly, in some embodiments, amplification of a target sequence is performed using a pair of primers and no additional primers or oligonucleotides are included in the amplification of the target sequence (e.g., the amplification reaction components comprise no additional primer pairs for a given target sequence, no nested primers, no bumper primers, no oligonucleotides other than the primers, no probes, and the like). In some embodiments, primers consist of a pair of primers, however, in some embodiments, an amplification reaction can include additional primer pairs for amplifying different target sequences, such as in a multiplex amplification. In some embodiments, primers consist of a pair of primers, however, in some embodiments, an amplification reaction can include additional primers, oligonucleotides or probes for a detection process that are not considered part of amplification. In some embodiments, primers are used in sets. An amplification primer set can include a pair of forward and reverse primers for a given target sequence. For multiplex amplification, primers that amplify a first target sequence are considered a primer set, and primers that amplify a second target sequence are considered a different primer set.

In some embodiments, amplification reaction components comprise a first primer (first oligonucleotide) complementary to a target sequence (e.g., a target nucleic acid) in a first strand (e.g., sense strand, forward strand) of a sample nucleic acid, and a second primer (second oligonucleotide) complementary to a target sequence in a second strand (e.g., antisense strand, reverse strand) of a sample nucleic acid. In some embodiments, a first primer (first oligonucleotide) comprises a first polynucleotide fully complementary to a target sequence in a first strand of sample nucleic acid, and a second primer (second oligonucleotide) comprises a second polynucleotide fully complementary to a target sequence in a second strand of sample nucleic acid. Fully complementary for a primer-target generally refers to a nucleotide sequence in a primer, where each base in order pairs with a correspondingly ordered base in a target sequence, and there are no gaps, additional sequences or unpaired bases within the sequence considered as fully complementary. Stated another way, fully complementary generally refers to all contiguous bases of a nucleotide sequence in a primer being complementary to corresponding contiguous bases of a nucleotide sequence in a target.

A primer can comprise one or more additional bases (e.g., at the 5' and/or 3' end, or within the primer) that do not add a functional feature to the primer. For example, additional sequences present in tailed primers or looped primers generally add a functional feature and would be excluded from primers in such embodiments. A primer, in some embodiments, can contain a modification such as one or more inosines, abasic sites, locked nucleic acids, minor groove binders, duplex stabilizers (e.g., acridine, spermidine), Tm modifiers or any modifier that changes the binding properties of the primer. A primer, in some embodiments, can contain a detectable molecule or entity (e.g., a fluorophore, radioisotope, colorimetric agent, particle, enzyme and the like).

### Polymerase

In some embodiments, amplification reactions comprise one or more polymerases. Polymerases are proteins capable of catalyzing the specific incorporation of nucleotides to extend a 3' hydroxyl terminus of a primer molecule, for example, an amplification primer described herein, against a nucleic acid target sequence (e.g., to which a primer is annealed). Polymerases may include, for example, thermophilic or hyperthermophilic polymerases that can have activity at an elevated reaction temperature (e.g., above 55°C, 60°C, 65°C, 70°C, 75°C, 80°C, 85°C, 80°C, 95°C, or 100°C). A hyperthermophilic polymerase may be referred to as a hyperthermophile polymerase. A polymerase may or may not have strand displacement capabilities. In some embodiments, a polymerase can incorporate about 1 to about 50 nucleotides in a single synthesis, for example, 5, 10, 15, 20, 25, 30, 35, 40, 45 or 50 nucleotides, or a number or a range between any two of these values, in a single synthesis.

Amplification reaction conditions can comprise one or more DNA polymerases, including but not limited to, 9°N DNA polymerase; 9°Nm^{™} DNA polymerase; Therminator^{™} DNA Polymerase; Therminator^{™} II DNA Polymerase; Therminator^{™} III DNA Polymerase; Therminator^{™} γ DNA Polymerase; Bst DNA polymerase; Bst DNA polymerase (large fragment); Phi29 DNA polymerase, DNA polymerase I (e.g., *E. coli*), DNA polymerase I, large (Klenow) fragment; Klenow fragment (3'-5' exo-); T4 DNA polymerase; T7 DNA polymerase; Deep VentR^{™} (exo-) DNA Polymerase; Deep VentR^{™} DNA Polymerase; DyNAzyme^{™} EXT DNA; DyNAzyme^{™} II Hot Start DNA Polymerase; Phusion^{™} High-Fidelity DNA Polymerase; VentR^{®} DNA Polymerase; VentR^{®} (exo-) DNA Polymerase; RepliPHI^{™} Phi29 DNA Polymerase; rBst DNA Polymerase, large fragment (IsoTherm^{™} DNA Polymerase); MasterAmp^{™} AmpliTherm^{™} DNA Polymerase; Tag DNA polymerase; Tth DNA polymerase; Tfl DNA polymerase; Tgo DNA polymerase; SP6 DNA polymerase; Tbr DNA polymerase; DNA polymerase Beta; and ThermoPhi DNA polymerase.

The DNA polymerase can be a hyperthermophile DNA polymerase, for example a hyperthermophile DNA polymerase that is thermostable at high temperatures, or a variant or a functional fragment thereof. For example, a hyperthermophile DNA polymerase may have a half-life of about 5 to 10 hours at 95°C and a half-life of about 1 to 3 hours at 100°C. In some embodiments, amplification reaction components comprise one or more hyperthermophile DNA polymerases from archaea, for example DNA polymerases from Thermococcus (e.g., *Thermococcaceaen archaean*)*, Pyrococcus,* Methanococcaceae, one or Methanococcus, Thermus, or a combination thereof. In some embodiments, amplification reaction conditions comprise one or more hyperthermophile DNA polymerases from *Thermus thermophiles.*

A functional fragment of a hyperthermophile DNA polymerase generally retains one or more functions of a full-length polymerase, for example, the capability to polymerize DNA (e.g., in an amplification reaction). A functional fragment can performs, for example, a function (e.g., polymerization of DNA in an amplification reaction) at a level that is at least about 50%, 75%, 80%, 85%, 90%, or 95% of the level of function for a full length polymerase. Levels of polymerase activity can be assessed, for example, using a detectable nucleic acid amplification method, such as a method for monitoring an amplification reaction as disclosed herein. In some embodiments, the hyperthermophile DNA polymerase comprises the amino acid sequence of SEQ ID NO: 1 or a functional fragment of SEQ ID NO: 1. In some embodiments, the hyperthermophile DNA polymerase comprising the amino acid sequence of SEQ ID NO: 2 or a functional fragment of SEQ ID NO: 2. In some embodiments, amplification reaction conditions comprise a polymerase comprising an amino acid sequence that is at least about 90% identical, 95% identical, or 99% identical to the amino acid sequence of SEQ ID NO: 1 or a functional fragment thereof. In some embodiments, amplification reaction conditions comprise a polymerase comprising an amino acid sequence that is at least about 90% identical, 95% identical, or 99% identical to the amino acid sequence of SEQ ID NO: 2 or a functional fragment thereof.

The polymerase can possess reverse transcription capabilities. In such instances, an amplification reaction can amplify RNA targets, for example, in a single step without the use of a separate reverse transcriptase. Non-limiting examples of polymerases that possess reverse transcriptase capabilities include Bst (large fragment), 9°N DNA polymerase, 9°Nm^{™} DNA polymerase, Therminator^{™}, Therminator^{™} II, and the like). In some embodiments, amplification reaction conditions comprise one or more separate reverse transcriptases. In some embodiments, more than one polymerase may be included in in an amplification reaction. For example, an amplification reaction can comprise a polymerase having reverse transcriptase activity and a second polymerase having no reverse transcriptase activity.

In some embodiments, one or more polymerases having exonuclease activity are used during amplification. In some embodiments, one or more polymerases having no or low exonuclease activity are used during amplification, for example a polymerase comprises one or more modifications (e.g., amino acid substitutions) that reduce or eliminate the exonuclease activity of the polymerase. For example, a modified polymerase having low exonuclease activity can have 10% or less exonuclease activity compared to an unmodified polymerase. For example, a modified polymerase having low exonuclease activity may have less than about 10%, 9%, 8%, 7%, 6%, 5%, 4%, 3%, 2%, or 1% exonuclease activity compared to an unmodified polymerase. In some embodiments, a polymerase has no or low 5' to 3' exonuclease activity, and/or no or low 3' to 5' exonuclease activity. In some embodiments, a polymerase has no or low single strand dependent exonuclease activity, and/or no or low double strand dependent exonuclease activity. Non limiting examples of the modifications that can reduce or eliminate exonuclease activity for a polymerase include one or more amino acid substitutions at one or more positions 141, 143 and 458 of SEQ ID NO: 1. An amino acid position corresponding to a position in SEQ ID NO: 1 may be identified, for example, by performing an amino acid sequence alignment. The modification(s) can include a substitution of the native amino acid at position 141 to an alanine, for example D141A; a substitution of the native amino acid at position 143 to an alanine, for example E143A; a substitution of the native amino acid at position 143 to an aspartate, for example E143D; a substitution of the native amino acid at position 485 to a leucine, for example A485L; or a combination thereof. In some embodiments, the modifications include one, two or more of D141A, E143A and A485L.

### Detection and Quantification

Methods disclosed herein can comprise detecting a signal generated from the quenchable label of the first quality control primer during the amplification reaction to determine generation of the extended duplex, wherein a decrease in the signal during the amplification reaction indicates generation of the extended duplex. In some embodiments, detecting the signal generated from the quenchable label of the first quality control primer during the amplification reaction can comprise detecting the signal at two or more different time points during the amplification reaction. The decrease in the signal during the amplification reaction can comprise a decrease over time during the amplification reaction. The decrease in the signal during the amplification reaction can comprise a decrease over a time period of about 10 minutes (e.g., about 1, 2, 3, 4, 5, 6, 7, 8, 9, 10 minute(s), or a number or a range between any two of these values) during the amplification reaction. The time period can be between about 3 minutes to about 12 minutes (e.g., about 3, 4, 5, 6, 7, 8, 9, 10, 11, 12 minute(s) or a number or a range between any two of these values) from the start of the amplification reaction. The method can comprise detecting the signal of the quenchable label of the first quality control primer before the amplification reaction, after the amplification reaction, or both. In some embodiments, detecting the signal generated from the quenchable label of the first quality control primer does not comprise using any probe. The method described herein can advantageously provide for reduced complexity in monitoring of amplification reactions without the need for additional primers and/or probes.

The methods described herein can comprise detecting and/or quantifying an amplification product (e.g., an extended duplex and/or target nucleic acid amplicon). An amplification product may be detected and/or quantified by any suitable detection and/or quantification method including, for example, any detection method or quantification method described herein. Non-limiting examples of detection and/or quantification methods include molecular beacon (e.g., real-time, endpoint), lateral flow, fluorescence resonance energy transfer (FRET), fluorescence polarization (FP), surface capture, 5' to 3' exonuclease hydrolysis probes (e.g., TAQMAN), intercalating/binding dyes, absorbance methods (e.g., colorimetric, turbidity), electrophoresis (e.g., gel electrophoresis, capillary electrophoresis), mass spectrometry, nucleic acid sequencing, digital amplification, a primer extension method (e.g., iPLEX^{™}), Molecular Inversion Probe (MIP) technology from Affymetrix, restriction fragment length polymorphism (RFLP analysis), allele specific oligonucleotide (ASO) analysis, methylation-specific PCR (MSPCR), pyrosequencing analysis, acycloprime analysis, Reverse dot blot, GeneChip microarrays, Dynamic allele-specific hybridization (DASH), Peptide nucleic acid (PNA) and locked nucleic acids (LNA) probes, AlphaScreen, SNPstream, genetic bit analysis (GBA), Multiplex minisequencing, SnaPshot, GOOD assay, Microarray miniseq, arrayed primer extension (APEX), Microarray primer extension, Tag arrays, Coded microspheres, Template-directed incorporation (TDI), colorimetric oligonucleotide ligation assay (OLA), sequence-coded OLA, microarray ligation, ligase chain reaction, padlock probes, invader assay, hybridization using at least one probe, hybridization using at least one fluorescently labeled probe, cloning and sequencing, the use of hybridization probes and quantitative real time polymerase chain reaction (QRT-PCR), nanopore sequencing, chips and combinations thereof. In some embodiments, detecting a nucleic acid amplification product comprises use of a real-time detection method (i.e., product is detected and/or continuously monitored during an amplification process). In some embodiments, detecting a nucleic acid amplification product comprises use of an endpoint detection method (i.e., product is detected after completing or stopping an amplification process). Nucleic acid detection methods can also employ the use of labeled nucleotides incorporated directly into a target sequence or into probes containing complementary sequences to a target. Such labels may be radioactive and/or fluorescent in nature and can be resolved in any of the manners discussed herein. In some embodiments, quantification of a nucleic acid amplification product may be achieved using one or more detection methods described below. In some embodiments, the detection method can be used in conjunction with a measurement of signal intensity, and/or generation of (or reference to) a standard curve and/or look-up table for quantification of a nucleic acid amplification product.

Detecting a nucleic acid amplification product (e.g., an extended duplex) can comprise use of fluorescence resonance energy transfer (FRET). FRET is an energy transfer mechanism between two chromophores: a donor and an acceptor molecule. Briefly, a donor fluorophore molecule is excited at a specific excitation wavelength. The subsequent emission from the donor molecule as it returns to its ground state may transfer excitation energy to the acceptor molecule through a long range dipole-dipole interaction. The emission intensity of the acceptor molecule can be monitored and is a function of the distance between the donor and the acceptor, the overlap of the donor emission spectrum and the acceptor absorption spectrum and the orientation of the donor emission dipole moment and the acceptor absorption dipole moment. FRET can be useful for quantifying molecular dynamics, for example, in DNA-DNA interactions as described for molecular beacons. For monitoring the production of a specific product, a probe can be labeled with a donor molecule on one end and an acceptor molecule on the other. Probe-target hybridization brings a change in the distance or orientation of the donor and acceptor and FRET change is observed. In some embodiments, the quenchable label is a donor molecule. In some embodiments, the acceptor molecule can be a quencher.

Detecting a nucleic acid amplification product (e.g., amplification products from amplification of target nucleic acids) can comprise use of molecular beacon, for example, hair-pin shaped oligonucleotides containing a fluorophore on one end and a quenching dye on the opposite end. The loop of the hair-pin can contain a probe sequence that is complementary to a target sequence and the stem is formed by annealing of complementary arm sequences located on either side of the probe sequence. A fluorophore and a quenching molecule can be covalently linked at opposite ends of each arm. Under conditions that prevent the oligonucleotides from hybridizing to its complementary target or when the molecular beacon is free in solution, the fluorescent and quenching molecules are proximal to one another preventing fluorescence resonance energy transfer (FRET). When the molecular beacon encounters a target molecule (e.g., a nucleic acid amplification product), hybridization can occur, and the loop structure is converted to a stable more rigid conformation causing separation of the fluorophore and quencher molecules leading to fluorescence. Due to the specificity of the probe, the generation of fluorescence generally is exclusively due to the synthesis of the intended amplified product. In some instances, a molecular beacon probe sequence hybridizes to a sequence in an amplification product that is identical to or complementary to a sequence in a target nucleic acid. In some instances, a molecular beacon probe sequence hybridizes to a sequence in an amplification product that is not identical to or complementary to a sequence in a target nucleic acid (e.g., hybridizes to a sequence added to an amplification product by way of a tailed amplification primer or ligation).

Detecting a nucleic acid amplification product can comprise using fluorescence polarization (FP), surface capture, 5' to 3' exonuclease hydrolysis probes (e.g., TAQMAN), intercalating and/or binding dyes, mass spectrometry, nucleic acid sequencing, digital amplification (e.g., digital PCR), and/or absorbance methods (e.g., colorimetric, turbidity). In some embodiments, detecting a nucleic acid amplification product comprises use of dyes that specifically stain nucleic acid. For example, intercalating dyes exhibit enhanced fluorescence upon binding to DNA or RNA. Dyes may include DNA or RNA intercalating fluorophores and may include for example, SYTO^{®} 82, acridine orange, ethidium bromide, Hoechst dyes, PicoGreen^{®}, propidium iodide, SYBR^{®} I (an asymmetrical cyanine dye), SYBR^{®} II, TOTO (a thiaxole orange dimer) and YOYO (an oxazole yellow dimer). Dyes provide an opportunity for increasing the sensitivity of nucleic acid detection when used in conjunction with various detection methods. For example, ethidium bromide may be used for staining DNA in agarose gels after gel electrophoresis; propidium iodide and Hoechst 33258 may be used in flow cytometry to determine DNA ploidy of cells; SYBR^{®} Green 1 may be used in the analysis of double-stranded DNA by capillary electrophoresis with laser induced fluorescence detection; and PicoGreen^{®} may be used to enhance the detection of double-stranded DNA after matched ion pair polynucleotide chromatography.

Provided herein are methods, kits, and reaction mixtures for evaluating, monitoring, observing, or tracking the progress of an amplification reaction. In another aspect, the methods, kits, and reaction mixtures disclosed herein can be used to determine amplification efficiency of a reaction. Non-limiting examples of factors that can interfere with amplification efficiency can include amplification inhibitors (e.g., hemoglobin, humic acid, fulvic acid, divalent cations, chelating molecules), absent and/or defective amplification reaction components, and enzymes and other proteins (e.g., nucleases).

The methods, kits, and reaction mixtures described herein can be used to determine the absence of amplification reaction components and/or the presence of defective amplification reaction components (e.g., quality). The quality of components may vary from batch to batch or over time. Thus, the quality of components can be controlled by evaluating the performance of the components in each batch using the methods described herein, or by evaluating the performance of the components in a batch over time. For example, the performance of two polymerases produced in a reference batch and a sample batch can be evaluated using the disclosed methods. The performance of the polymerase in the sample batch can be compared with that of polymerase in the reference batch to determine whether the polymerase in the sample batch is suitable for use. Alternatively, the activity of a polymerase can be compared before and after the lapse of a predetermined time to determine whether the activity of the polymerase decreases over time. In some embodiments, the disclosed methods can determine the shelf life of one or more amplification components. For example, the activity of the polymerase is evaluated at a predetermined time interval from the initial production. If the activity falls below a predetermined level after a certain period of time, the time can be determined as the shelf life of the polymerase.

In some embodiments, the methods, kits, and reaction mixtures disclosed herein can be used (e.g., as an internal control) to monitor amplification efficiency, for example the presence of amplification inhibitors which interferes with amplification efficiency. In general, an internal control is used to monitor the inhibitory activity of a sample (e.g., the presence of amplification inhibitors). An internal control can include: (i) a template irrelevant to a target nucleic acid sequence suspected of being contained in a sample and (ii) primers and probes for amplifying and detecting the template. It is advantageous that the amplification efficiency of the internal control is not affected by the amplification of the target nucleic add sequence contained in the sample, and vice versa. The amplification of an internal control indicates that the sample has no inhibitory activity against the amplification reaction, i.e., the sample contains no substances that inhibit the amplification reaction.

The first and second quality control primers described herein can replace the components of a conventional internal control, i.e., a template, primer pairs and a probe. The first and second quality control primers described herein can be further added to an amplification reaction of a target nucleic acid sequence in a sample, so that amplification of the duplex formed by hybridization between the 3' overlapping regions of the first and second quality control primers can occur simultaneously with amplification of the target nucleic add sequence. The production of an extended duplex can allow the determination of whether an inhibitory substance against the amplification reaction is present in a sample.

The methods, kits, and reaction mixtures described herein can be used as a quantification standard. For example, in a quantitative real-time PCR reaction, quantification of a target nucleic acid sequence is accomplished by performing amplification reactions using a dilution series of a known standard to obtain a standard curve in which a log value of the initial amount of the target nucleic acid sequence is plotted against a Ct value, and then comparing a Ct value obtained from an unknown sample with the standard curve to calculate the amount of the target nucleic acid sequence in the sample. The first and second quality control primers can be advantageously used in place of the standard.

### Target nucleic acid

As used herein, a target nucleic acid is a nucleic acid of interest that is subject to amplification in the amplification reaction being evaluated and/or monitored using the quality control methods and compositions disclosed herein. The terms "nucleic acid" and "nucleic acid molecule" are used interchangeably herein. The terms refer to nucleic acids of any composition, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, and/or DNA or RNA analogs (e.g., containing base analogs, sugar analogs and/or a non-native backbone and the like), RNA/DNA hybrids and polyamide nucleic acids (PNAs), all of which can be in single- or double-stranded form, and unless otherwise limited, can encompass known analogs of natural nucleotides that can function in a similar manner as naturally occurring nucleotides. A nucleic acid can be, or can be from, a plasmid, phage, autonomously replicating sequence (ARS), centromere, artificial chromosome, chromosome, or other nucleic acid able to replicate or be replicated in vitro or in a host cell, a cell, a cell nucleus, a mitochondria, or cytoplasm of a cell. Unless specifically limited, the term encompasses nucleic acids containing known analogs of natural nucleotides that have similar binding properties as the reference nucleic acid and are metabolized in a manner similar to naturally occurring nucleotides.

Target nucleic acids may be referred to as target sequences, target polynucleotides, and/or target polynucleotide sequences, and may include double-stranded and single-stranded nucleic acid molecules. Target nucleic acid can be, for example, DNA or RNA. A target sequence may refer to either the sense or antisense strand of a nucleic acid sequence, and also may refer to sequences as they exist on target nucleic acids, amplified copies, or amplification products, of the original target sequence. A target sequence can be a subsequence within a larger polynucleotide. For example, a target sequence may be a short sequence (e.g., 20 to 50 bases) within a nucleic acid fragment, a chromosome, a plasmid, that is targeted for amplification. In some embodiments, a target sequence refers to a sequence in a target nucleic acid that is complementary to an oligonucleotide (e.g., primer) used for amplifying a nucleic acid. Thus, a target sequence may refer to the entire sequence targeted for amplification or may refer to a subsequence in the target nucleic acid where an oligonucleotide binds. An amplification product can be a larger molecule that comprises the target sequence, as well as at least one other sequence, or other nucleotides. In some embodiments, an amplification product is about the same length as the target sequence, or is exactly the same length as the target sequence. In some embodiments, an amplification product comprises the target sequence. In some embodiments, an amplification product consists of the target sequence.

Target nucleic acid can include, for example, genomic nucleic acid, plasmid nucleic acid, mitochondrial nucleic acid, cellular nucleic acid, extracellular nucleic acid, bacterial nucleic acid and viral nucleic acid. In some embodiments, target nucleic acid may include genomic DNA, chromosomal DNA, plasmid DNA, mitochondrial DNA, a gene, any type of cellular RNA, messenger RNA, bacterial RNA, viral RNA or a synthetic oligonucleotide. Genomic nucleic acid may include any nucleic acid from any genome, for example, including animal, plant, insect, viral and bacterial genomes, including, for example, genomes present in spores. In some embodiments, genomic target nucleic acid may be within a particular genomic locus or a plurality of genomic loci. A genomic locus may include any or a combination of open reading frame DNA, non-transcribed DNA, intronic sequences, extronic sequences, promoter sequences, enhancer sequences, flanking sequences, or any sequences considered associated with a given genomic locus. Target nucleic acid can include microRNAs (miRNAs) or short interfering RNAs (siRNAs).

Nucleic acid can be obtained from any suitable biological specimen or sample, and often is isolated from a sample obtained from a subject. A subject can be any living or non-living organism, including but not limited to a human, a non-human animal, a plant, a bacterium, a fungus, a virus, or a protist. Any human or non-human animal can be selected, including but not limited to mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female, and a subject may be any age (e.g., an embryo, a fetus, infant, child, adult).

A sample or test sample can be any specimen that is isolated or obtained from a subject or part thereof, including, without limitation, blood or a blood product (e.g., serum, plasma, or the like), umbilical cord blood, bone marrow, chorionic villi, amniotic fluid, cerebrospinal fluid, spinal fluid, lavage fluid (e.g., bronchoalveolar, gastric, peritoneal, ductal, ear, arthroscopic), biopsy sample, celocentesis sample, cells (e.g., blood cells) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, hard tissues (e.g., liver, spleen, kidney, lung, or ovary), or combinations thereof. The term blood encompasses whole blood, blood product or any fraction of blood, such as serum, plasma, buffy coat, or the like as conventionally defined. Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow.

A sample or test sample can include samples containing spores, viruses, cells, nucleic acid from prokaryotes or eukaryotes, or any free nucleic acid. For example, a method described herein may be used for detecting nucleic acid on the outside of spores (e.g., without the need for lysis). A sample can be isolated from any material suspected of containing a target sequence, such as from a subject described above. In some embodiments, a target sequence is present in air, plant, soil, or other materials suspected of containing biological organisms.

Any suitable method can be used for isolating, extracting and/or purifying nucleic acid from a biological sample, non-limiting examples of which include methods of DNA preparation in the art, and various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep^{™} Blood DNA Isolation Kit (Promega, Madison, Wis.), GFX^{™} Genomic Blood DNA Purification Kit (Amersham, Piscataway, N.J.), and the like or combinations thereof.

A sample can comprise sample nucleic acids (e.g., a plurality of sample nucleic acids). The term "plurality" is used herein to mean two or more. Thus, in some embodiments, a sample includes two or more (e.g., 3 or more, 5 or more, 10 or more, 20 or more, 50 or more, 100 or more, 500 or more, 1,000 or more, or 5,000 or more) sample nucleic acids (e.g., DNAs/RNAs). A disclosed method can be used as a very sensitive way to detect a target nucleic acid present in a sample (e.g., in a complex mixture of nucleic acids such as DNAs/RNAs). In some embodiments the sample includes 5, 10, 20, 25, 50, 100, 500, 10³, 5×10³, 10⁴, 5×10⁴, 10⁵, 5×10⁵, 10⁶, or 10⁷, 50, or more, DNAs/RNAs that differ from one another in sequence. In some embodiments, the sample includes DNAs/RNAs from a cell (e.g., a eukaryotic cell, a mammalian cell, or a human cell) or a cell lysate (e.g., a eukaryotic cell lysate, a mammalian cell lysate, a human cell lysate, a prokaryotic cell lysate, a plant cell lysate, or the like).

The term "sample" is used here shall be given its ordinary meaning and shall include any sample that includes RNA and/or DNA (e.g., in order to determine whether a target DNA and/or target RNA is present among a population of RNAs and/or DNAs). The sample can be derived from any source, e.g., the sample can be a synthetic combination of purified DNAs and/or RNAs; the sample can be a cell lysate, an DNA/RNA-enriched cell lysate, or DNAs/RNAs isolated and/or purified from a cell lysate. The sample can be from a patient (e.g., for the purpose of diagnosis). The sample can be from permeabilized cells, crosslinked cells, tissue sections, or combination thereof. The sample can be from tissues prepared by crosslinking followed by delipidation and adjustment to make a uniform refractive index. A sample can include a target nucleic acid (e.g., target DNA/RNA) and a plurality of non-target DNAs/RNAs. In some embodiments, the target DNA/RNA is present in the sample at one copy per 10, 20, 25, 50, 100, 500, 10³, 5×10³, 10⁴, 5×10⁴, 10⁵, 5×10⁵, 10⁶, or 10⁷, non-target DNAs/RNAs.

A sample with respect to a patient encompasses blood and other liquid samples of biological origin, solid tissue samples such as a biopsy specimen or tissue cultures or cells derived therefrom and the progeny thereof, as well as samples that have been manipulated in any way after their procurement (such as by treatment with reagents); washed; or enriched for certain cell populations (e.g., cancer cells) or particular types of molecules (e.g., RNAs). A sample can comprise, or be, a biological sample including but not limited to a clinical sample such as blood, plasma, serum, aspirate, cerebral spinal fluid (CSF), and also includes tissue obtained by surgical resection, tissue obtained by biopsy, cells in culture, cell supernatants, cell lysates, tissue samples, organs, bone marrow, and the like. A biological sample can comprise biological fluids derived therefrom (e.g., cancerous cell, infected cell, etc.), e.g., a sample comprising RNAs that is obtained from such cells (e.g., a cell lysate or other cell extract comprising RNAs).

The source of the sample can be a (or is suspected of being a) diseased cell, fluid, tissue, or organ; or a normal (non-diseased) cell, fluid, tissue, or organ. In some embodiments, the source of the sample is a (or is suspected of being a) pathogen-infected cell, tissue, or organ. For example, the source of a sample can be an individual who may or may not be infected-and the sample can be any biological sample (e.g., blood, saliva, biopsy, plasma, serum, bronchoalveolar lavage, sputum, a fecal sample, cerebrospinal fluid, a fine needle aspirate, a swab sample (e.g., a buccal swab, a cervical swab, a nasal swab), interstitial fluid, synovial fluid, nasal discharge, tears, buffy coat, a mucous membrane sample, an epithelial cell sample (e.g., epithelial cell scraping), etc.) collected from the individual. The sample can be a cell-free liquid sample or a liquid sample that comprise cells. Pathogens can be viruses, fungi, protozoa, *Plasmodium* parasites, *Toxoplasma* parasites, *Schistosoma* parasites, and the like. "Helminths" include roundworms, heartworms, and phytophagous nematodes (Nematoda), flukes (Tematoda), Acanthocephala, and tapeworms (Cestoda). Protozoan infections include infections from Giardia spp., *Trichomonas* spp., African trypanosomiasis, amoebic dysentery, babesiosis, balantidial dysentery, Chaga's disease, coccidiosis, malaria and toxoplasmosis. Examples of pathogens such as parasitic/protozoan pathogens include, but are not limited to: *Plasmodium falciparum, Plasmodium vivax, Trypanosoma cruzi* and *Toxoplasma gondii.* Fungal pathogens include, but are not limited to: *Cryptococcus neoformans, Histoplasma capsulatum, Coccidioides immitis, Blastomyces dermatitidis, Chlamydia trachomatis,* and *Candida albicans.* Pathogenic viruses include, e.g., immunodeficiency virus (e.g., HIV); influenza virus; dengue; West Nile virus; herpes virus; yellow fever virus; Hepatitis C virus; Hepatitis A virus; Hepatitis B virus; papillomavirus; and the like. Pathogenic viruses can include DNA viruses such as: a papovavirus (e.g., HPV, polyomavirus); a hepadnavirus; a herpesvirus (e.g., HSV (e.g., HSV I, HSV II), varicella zoster virus (VZV), epstein-barr virus (EBV), cytomegalovirus (CMV), herpes lymphotropic virus, *Pityriasis Rosea,* kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like. Non-limiting examples of pathogens include *Mycobacterium tuberculosis, Streptococcus agalactiae,* methicillin-resistant *Staphylococcus aureus, Legionella pneumophila, Streptococcus pyogenes, Escherichia coli, Neisseria gonorrhoeae, Neisseria meningitidis, Pneumococcus, Cryptococcus neoformans, Histoplasma capsulatum, Hemophilus influenzae B, Treponema pallidum,* Lyme disease spirochetes, *Pseudomonas aeruginosa, Mycobacterium leprae, Brucella abortus,* rabies virus, human serum parvo-like virus, respiratory syncytial virus, measles virus, adenovirus, human T-cell leukemia viruses, murine leukemia virus, mumps virus, vesicular stomatitis virus, Sindbis virus, lymphocytic choriomeningitis virus, wart virus, blue tongue virus, Sendai virus, feline leukemia virus, reovirus, polio virus, simian virus 40, mouse mammary tumor virus, dengue virus, rubella virus, *Toxoplasma gondii, Trypanosoma rangeli, Trypanosoma cruzi, Trypanosoma rhodesiense, Trypanosoma brucei, Schistosoma mansoni, Schistosoma japonicum, Babesia bovis, Eimeria tenella, Onchocerca volvulus, Leishmania tropica, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium* and *M. pneumoniae.*

### Kits

Disclosed herein include kits for evaluating, monitoring, observing, or tracking the progress of an amplification reaction. The kit can comprise a first quality control primer and a second quality control primer each comprising a 3' overlapping region capable of hybridizing to each other, wherein the first quality control primer comprises a quenchable label. In some embodiments, each of the first quality control primer and the second quality control primer is no more than 35 nucleotides in length.

The quenchable label can be a fluorophore. The quenchable label can be outside of the 3' overlapping region of the first quality control primer. The quenchable label can be at 5' terminus of the first quality control primer. The quenchable label can be in the 3' overlapping region of the first quality control primer. The second quality control primer can comprise a quencher. The quencher can be outside of the 3' overlapping region of the second quality control primer. The quencher can be at 5' terminus of the second quality control primer. The quencher can be in the 3' overlapping region of the second quality control primer.

The first quality control primer, the second quality control primer, or both, can comprise one or more modified nucleotides. Each of the 3' overlapping regions of the first and second quality control primers can comprise one or more modified nucleotides. The one or more modified nucleotides can comprise a spacer, an a-basic site, an un-methylated RNA base, a 2'-O-methylated nucleotide, and any combination thereof. At least one of the one or more modified nucleotides can be a 2'-O-methylated nucleotide. The first quality control primer, the second quality control primer, or both, can comprise one or more polymerase stoppers. In some embodiments, each of the 3' overlapping regions of the first and second quality control primers can comprise one or more polymerase stoppers. In some embodiments, at least one of the one or more polymerase stoppers can be a 2'-O-methylated nucleotide.

The 3' overlapping region of the first quality control primer can be complementary to the 3' overlapping region of the second quality control primer. The 3' overlapping region of the first quality control primer can be fully complementary to the 3' overlapping region of the second quality control primer. In some embodiments, the 3' overlapping region of the first quality control primer and the 3' overlapping region of the second quality control primer have the same length. One or more of the 3' overlapping region of the first quality control primer and the 3' overlapping region of the second quality control primer can be about 2 to about 10 nucleotides in length. The 3' overlapping region of the first and second quality control primers can be 4 or 5 nucleotides in length.

The kit can comprise one or more of an enzyme having a hyperthermophile polymerase activity, dNTPs, and a buffering agent. The enzyme having a hyperthermophile polymerase activity has an amino acid sequence that can be at least about 90% or at least about 95% identical to the amino acid sequence of SEQ ID NO: 1 or a functional fragment thereof. The enzyme having a hyperthermophile polymerase activity can comprise the amino acid sequence of SEQ ID NO: 1. Kits can further comprise, for example, modified nucleotides, vessels, cuvettes or other containers used for the reaction, or a vial of water or buffer for rehydrating lyophilized or heat-dried components. The buffer used can, for example, be appropriate for both polymerase and primer annealing activity. The kit can comprise one or more additional primers and/or one or more probes specific to a target nucleic acid.

Kits can also comprise one or more of the components in any number of separate vessels, chambers, containers, packets, tubes, vials, microtiter plates and the like, or the components can be combined in various combinations in such containers. Components of the kit can, for example, be present in one or more containers. In some embodiments, all of the components are provided in one container. In some embodiments, the enzymes (e.g., polymerase(s) and/or reverse transcriptase(s)) can be provided in a separate container from the primers. The components can, for example, be lyophilized, heat dried, freeze dried, or in a stable buffer. In some embodiments, polymerase(s) and/or reverse transcriptase(s) are in lyophilized form or heat dried form in a single container, and the primers are either lyophilized, heat dried, freeze dried, or in buffer, in a different container. In some embodiments, polymerase(s) and/or reverse transcriptase(s), and the primers are, in lyophilized form or heat dried form, in a single container.

The compositions described herein (e.g., dried composition) can be provided in a "dry form," or in a form not suspended in liquid medium. The "dry form" of the compositions can include dry powders, lyophilized compositions, spray-dried, or precipitated compositions. The "dry form" compositions can include one or more lyoprotectants, such as sugars and their corresponding sugar alcohols, such as sucrose, lactose, trehalose, dextran, erythritol, arabitol, xylitol, sorbitol, and mannitol; amino acids, such as arginine or histidine; lyotropic salts, such as MgSO₄; polyols, such as propylene glycol, glycerol, poly(ethylene glycol), or polypropylene glycol); and combinations thereof. Additional exemplary lyoprotectants include gelatin, dextrins, modified starch, and carboxymethyl cellulose. As used herein, the terms "lyophilization," "lyophilized," and "freeze-dried" refer to a process by which the material to be dried is first frozen and then the ice or frozen solvent is removed by sublimation in a vacuum environment. "Lyophilisate" refers to a lyophilized substance. As disclosed herein, the dried composition can comprise one or more additives and one or more amplification reagents.

The dried composition can be frozen or lyophilized or spray dried. The dried composition can be heat dried. The dried composition can comprise one or more additives (e.g., a polymer, a sugar or sugar alcohol). The sugar or sugar alcohol can comprise sucrose, lactose, trehalose, dextran, erythritol, arabitol, xylitol, sorbitol, mannitol, or any combination thereof. The polymer can comprise polyethylene glycol, dextran, polyvinyl alcohol, hydroxypropyl methylcellulose, gelatin, polyvinylpyrrolidone, hydroxyethyl cellulose, Ficoll, albumin, a polypeptide, a collagen peptide, or any combination thereof. The one or more additives can comprise one or more amino acids. The one or more additives can comprise Tween 80, Tween 20 and/or Triton X-100. In some embodiments, the one or more additives help lyophilization of the reaction compositions and/or the dissolution of dried pellets. The one or more additives can comprise a nonionic detergent at a concentration of about 0.01% in the dried composition (e.g., dried pellet).

The frozen or lyophilized or spray dried or heat dried composition or the aqueous composition for preparing the frozen or lyophilized or spray dried composition may comprise one or more of the following: (i) Non-aqueous solvents such as ethylene glycol, glycerol, dimethylsulphoxide, and dimethylformamide. (ii) Surfactants such as Tween 80, Brij 35, Brij 30, Lubrol-px, Triton X-10; Pluronic F127 (polyoxyethylene-polyoxypropylene copolymer) also known as poloxamer, poloxamine, and SDS. (iii) Dissacharides such as trehalose, sucrose, lactose, and maltose. (iv) Polymers (which may have different MWs) such as polyethylene glycol, dextran, polyvinyl alcohol), hydroxypropyl methylcellulose, gelatin, polyvinylpyrrolidone, hydroxyethyl cellulose, Ficoll, and albumin. (v) Amino acids such as glycine, proline, 4-hydroxyproline, L-serine, glutamate, alanine, lysine, sarcosine, and gamma-aminobutyric acid. The first quality control primer and the second quality control primer can be in a lyophilized or freeze-dried form. The one or more of an enzyme having a hyperthermophile polymerase activity, dNTPs, and a buffering agent can be in a lyophilized or freeze-dried form.

Kits can comprise instructions for performing one or more methods described herein and/or a description of one or more components described herein. Instructions and/or descriptions can be in printed form and can be included in a kit insert. The kit also can include a written description of an internet location that provides such instructions or descriptions. In some embodiments, the kit comprises reagents used for detection methods, for example, reagents used for FRET, lateral flow devices, dipsticks, fluorescent dye, colloidal gold particles, latex particles, a molecular beacon, or polystyrene beads.

### EXAMPLES

Some aspects of the embodiments discussed above are disclosed in further detail in the following examples, which are not in any way intended to limit the scope of the present disclosure.

### Example 1

### DIMER internal control

This example describes monitoring an amplification reaction.

A forward and a reverse IC primers were used in an amplification reaction. The forward IC primer has an fluorophore associated at its 5' end, and the reverse IC primer is associated with a quencher at the 5' end (FIG. 1). At the beginning of the reaction, the forward and the reverse IC primers are separate in solution, and thus the fluorophores on the forward IC primer are fully emitting (generating high signal) (FIG. 2). As the archaeal polymerase amplification (APA) reaction progresses, the Dimer-IC amplicon (e.g., extended duplex) brings the fluorophore and quencher into close proximity, so that FRET quenching can occur which causes a decrease in signal. Therefore, an "inverted" amplification curve is ultimately produced, as shown in FIG. 2.

FIG. 3A-FIG. 3B show an inverted amplification curve observed using a Dimer-IC system disclosed herein:
Forward Primer (FP1) + Reverse Primer (RP3) (5bp overlap),
Forward Primer (FP1) + Reverse Primer (RP2) (4bp overlap),
Forward Primer only control (FP1 only) (no amplification expected).

The intercalating dye signal (run in the same reaction) demonstrated that amplification occurred as intended.

FIG. 4 shows a pair of quality control primers: (1) a forward quality control primer FP3 having a sequence of SEQ ID NO: 3 and an internal 2'OM modification at nucleotide position 10 and (2) a reverse quality control primer RP4 having the sequence of SEQ ID NO: 5 and an internal 2'OM modification at nucleotide position 9.

In some instances, unintended primer extension by a quality primer with other oligos can be prevented via the inclusion of one or more nucleotides with 2'OM modifications in the quality control primer. As shown in FIG. 5A, nucleic acid extension from an unintended hybridization that occurs at nucleotide positions 12-13 of the forward quality control primer (FP3) with an non-IC-primer oligonucleotide is arrested due to the inability of DNA polymerase to read through the 2'-OM modified nucleotide at position 10 in FP3. In some embodiments, unintended primer extension (for example, on human gDNA) and subsequent amplification via unintended reverse priming (via one of the other primers present in the reaction) can be likewise prevented (or significantly reduced) via the inclusion of modified nucleotide(s) (e.g.., nucleotides with 2'OM modification) at one or more positions of the quality control primers. As shown in FIG. 5B, the inability for DNA polymerase to read through nucleotide position 10 in the depicted forward quality control primer FP3 renders this quality control primer much less vulnerable to non-specific amplification.

As shown in FIG. 6A-FIG. 6C, the quenchable label and quencher can be placed at various regions of the quality control primers, for example outside and/or inside the 3' overlapping region of the first and second quality control primers. Without being bound by any particular theory, it is expected that placing a quenchable label in the 3' overlapping region of the forward quality control primer and placing a quencher in the 3' overlapping region of the reverse quality control primer can result in contact-quenching (as opposed to FRET quenching), and can yield a greater change in signal (FIG. 6B).

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.,* bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A method for monitoring an amplification reaction, comprising
(a) providing a first quality control primer and a second quality control primer each comprising a 3' overlapping region capable of hybridizing to each other, wherein each of the first quality control primer and the second quality control primer is no more than 35 nucleotides in length, wherein the first quality control primer comprises a quenchable label, wherein the quenchable label is a fluorophore, and wherein the second quality control primer comprises a quencher;
(b) contacting the first quality control primer and the second quality control primer, thereby forming a duplex via hybridization between the 3' overlapping regions of the first and second quality control primers;
(c) subjecting the duplex to an amplification condition, thereby generating an extended duplex; and
(d) detecting a signal generated from the quenchable label of the first quality control primer during the amplification reaction to determine generation of the extended duplex, wherein a decrease in the signal during the amplification reaction indicates generation of the extended duplex.

2. The method of claim 1, wherein:
a. the quenchable label is:
i. outside of the 3' overlapping region of the first quality control primer, optionally wherein the quenchable label is at 5' terminus of the first quality control primer; or
ii. in the 3' overlapping region of the first quality control primer; and/or
b. the quencher is:
i. outside of the 3' overlapping region of the second quality control primer, optionally wherein the quencher is at 5' terminus of the second quality control primer; or
ii. in the 3' overlapping region of the second quality control primer.

3. The method of any one of claims 1 or 2, wherein:
a. the first quality control primer, the second quality control primer, or both, comprises:
i. one or more modified nucleotides, optionally wherein:
1. each of the 3' overlapping regions of the first and second quality control primers comprises one or more modified nucleotides; and/or
2. the one or more modified nucleotides comprise a spacer, an a-basic site, an un-methylated RNA base, a 2'-O-methylated nucleotide, and any combination thereof; and/or
3. at least one of the one or more modified nucleotides is a 2'-O-methylated nucleotide; or
ii. one or more polymerase stoppers, optionally wherein:
1. each of the 3' overlapping regions of the first and second quality control primers comprises one or more polymerase stoppers; and/or
2. at least one of the one or more polymerase stoppers is a 2'-O-methylated nucleotide; and/or
b. the 3' overlapping region of the first quality control primer is complementary to the 3' overlapping region of the second quality control primer, optionally wherein the 3' overlapping region of the first quality control primer is fully complementary to the 3' overlapping region of the second quality control primer; and/or
c. the 3' overlapping region of the first quality control primer and the 3' overlapping region of the second quality control primer have the same length; and/or
d. one or more of the 3' overlapping region of the first quality control primer and the 3' overlapping region of the second quality control primer is about 2 to about 10 nucleotides in length; and/or
e. the 3' overlapping region of the first and second quality control primers is 4 or 5 nucleotides in length.

4. The method of any one of claims 1-3, wherein:
a. (b) contacting the first quality control primer and the second quality control primer is carried out under the amplification condition; and/or
b. (d) detecting the signal generated from the quenchable label of the first quality control primer during the amplification reaction comprises detecting the signal at two or more different time points during the amplification reaction; and/or
c. the decrease in the signal during the amplification reaction comprises a decrease over time during the amplification reaction, optionally wherein the decrease in the signal during the amplification reaction comprises a decrease over a time period of:
i. about 10 minutes during the amplification reaction; or
ii. about 3 minutes to about 12 minutes from the start of the amplification reaction; and/or
d. the method comprises detecting the signal of the quenchable label of the first quality control primer before the amplification reaction, after the amplification reaction, or both.

5. The method of any one of claims 1-4, wherein:
a. the amplification reaction is a real-time amplification reaction; and/or
b. the amplification reaction:
i. is a PCR reaction; or
ii. comprises one or more of the following: archaeal polymerase amplification (APA), loop-mediated isothermal amplification (LAMP), helicase-dependent amplification (HDA), recombinase polymerase amplification (RPA), strand displacement amplification (SDA), nucleic acid sequence-based amplification (NASBA), transcription mediated amplification (TMA), nicking enzyme amplification reaction (NEAR), rolling circle amplification (RCA), multiple displacement amplification (MDA), Ramification (RAM), circular helicase-dependent amplification (cHDA), single primer isothermal amplification (SPIA), signal mediated amplification of RNA technology (SMART), selfsustained sequence replication (3SR), genome exponential amplification reaction (GEAR) and isothermal multiple displacement amplification (IMDA); or
iii. is an isothermal amplification reaction, optionally wherein:
1. the amplification condition comprises one or more of an enzyme having a hyperthermophile polymerase activity, dNTPs, and a buffering agent, further optionally wherein the enzyme having a hyperthermophile polymerase activity has an amino acid sequence that is at least about 90% identical to the amino acid sequence of SEQ ID NO: 1 or a functional fragment thereof, and optionally the enzyme having a hyperthermophile polymerase activity comprises the amino acid sequence of SEQ ID NO: 1; and/or
2. the isothermal amplification reaction comprises a constant temperature of about 30°C to about 72°C, and optionally the isothermal amplification reaction comprises a constant temperature of about 67°C; and/or
3. the isothermal amplification reaction is performed for a period of about 5 minutes to about 60 minutes; and/or
4. the isothermal amplification reaction is performed in a helicase-free, single-stranded binding protein-free, cleavage agent-free, and recombinase-free, isothermal amplification condition.

6. The method of any one of claims 1-5, wherein:
a. detecting the signal generated from the quenchable label of the first quality control primer does not comprise using any probe; and/or
b. (c) subjecting the duplex to the amplification condition comprises subjecting a target nucleic acid and one or more additional primers and/or one or more probes specific to the target nucleic acid to the amplification condition, optionally wherein:
i. the first quality control primer does not hybridize to the target nucleic acid under the amplification condition; and/or
ii. the second quality control primer does not hybridize to the target nucleic acid under the amplification condition.

7. The method of any one of claims 1-6, wherein:
a. the method does not comprise using any template nucleic acid capable of hybridizing to the first quality control primer; and/or
b. the method does not comprise using any template nucleic acid capable of hybridizing to the second quality control primer.

8. A kit for monitoring an amplification reaction, comprising
a first quality control primer and a second quality control primer each comprising a 3' overlapping region capable of hybridizing to each other, wherein each of the first quality control primer and the second quality control primer is no more than 35 nucleotides in length, wherein the first quality control primer comprises a quenchable label, and wherein the second quality control primer comprises a quencher.

9. The kit of claim 8, wherein:
a. the quenchable label is a fluorophore; and/or
b. the quenchable label is:
i. outside of the 3' overlapping region of the first quality control primer, optionally wherein the quenchable label is at 5' terminus of the first quality control primer; or
ii. the quenchable label is in the 3' overlapping region of the first quality control primer; and/or
c. the quencher is:
i. outside of the 3' overlapping region of the second quality control primer, optionally wherein the quencher is at 5' terminus of the second quality control primer; or
ii. the quencher is in the 3' overlapping region of the second quality control primer.

10. The kit of any one of claims 8 or 9, wherein:
a. the first quality control primer, the second quality control primer, or both, comprises:
i. one or more modified nucleotides, optionally wherein:
1. each of the 3' overlapping regions of the first and second quality control primers comprises one or more modified nucleotides; and/or
2. the one or more modified nucleotides comprise a spacer, an a-basic site, an un-methylated RNA base, a 2'-O-methylated nucleotide, and any combination thereof; or
ii. one or more polymerase stoppers, optionally wherein:
1. each of the 3' overlapping regions of the first and second quality control primers comprises one or more polymerase stoppers; and/or
2. at least one of the one or more polymerase stoppers is a 2'-O-methylated nucleotide; and/or
b. the 3' overlapping region of the first quality control primer is complementary to the 3' overlapping region of the second quality control primer, optionally wherein the 3' overlapping region of the first quality control primer is fully complementary to the 3' overlapping region of the second quality control primer; and/or
c. the 3' overlapping region of the first quality control primer and the 3' overlapping region of the second quality control primer have the same length; and/or
d. one or more of the 3' overlapping region of the first quality control primer and the 3' overlapping region of the second quality control primer is about 2 to about 10 nucleotides in length; and/or
e. the 3' overlapping region of the first and second quality control primers is 4 or 5 nucleotides in length.

11. The kit of any one of claims 8-10, comprising one or more of an enzyme having a hyperthermophile polymerase activity, dNTPs, and a buffering agent, optionally wherein the enzyme having a hyperthermophile polymerase activity has an amino acid sequence that is at least about 90% to the amino acid sequence of SEQ ID NO: 1 or a functional fragment thereof, optionally the enzyme having a hyperthermophile polymerase activity comprises the amino acid sequence of SEQ ID NO: 1.

12. The kit of any one of claims 8-11, wherein:
a. the first quality control primer and the second quality control primer are in a lyophilized or freeze-dried form, optionally wherein the one or more of an enzyme having a hyperthermophile polymerase activity, dNTPs, and a buffering agent are in a lyophilized or freeze-dried form; and/or
b. the kit further comprises one or more additional primers and/or one or more probes specific to a target nucleic acid.

13. A reaction mixture, comprising
a first quality control primer and a second quality control primer each comprising a 3' overlapping region capable of hybridizing to each other, wherein each of the first quality control primer and the second quality control primer is no more than 35 nucleotides in length, wherein the first quality control primer comprises a quenchable label, and wherein the second quality control primer comprises a quencher;
a target nucleic acid; and
one or more additional primers and/or one or more probes specific to the target nucleic acid.

14. The reaction mixture of claim 13, wherein:
a. the reaction mixture comprises a duplex formed by hybridization between the 3' overlapping regions of the first and second quality control primers; and/or
b. the reaction mixture comprises one or more of an enzyme having a polymerase activity, dNTPs, and a buffering agent; and optionally the enzyme is an enzyme having a hyperthermophile polymerase activity, optionally wherein the enzyme having a hyperthermophile polymerase activity has an amino acid sequence that is at least about 90% identical to the amino acid sequence of SEQ ID NO: 1 or a functional fragment thereof, and optionally the enzyme having a hyperthermophile polymerase activity comprises the amino acid sequence of SEQ ID NO: 1.

15. The reaction mixture of any one of claims 13 or 14, wherein:
a. the first quality control primer and the second quality control primer are not capable of hybridizing to the target nucleic acid; and/or
b. the one or more additional primers and/or one or more probes are not capable of hybridizing to the first quality control primer, the second quality control primer, or both.

## Patentansprüche

1. Verfahren zur Überprüfung einer Amplifikationsreaktion, umfassend
(a) Bereitstellen eines ersten Qualitätskontrollprimers und eines zweiten Qualitätskontrollprimers, die jeweils einen 3'-Überlappungsbereich aufweisen, der in der Lage ist, sie miteinander zu hybridisieren, wobei der erste Qualitätskontrollprimer und der zweite Qualitätskontrollprimer jeweils nicht länger als 35 Nukleotide sind, wobei der erste Qualitätskontrollprimer eine deaktivierbare Markierung umfasst, wobei es sich bei der deaktivierbaren Markierung um ein Fluorophor handelt und wobei der zweite Qualitätskontrollprimer ein Deaktivierungsmittel umfasst;
(b) Inkontaktbringen des ersten Qualitätskontrollprimers und des zweiten Qualitätskontrollprimers, wobei durch Hybridisierung zwischen den 3'-Überlappungsbereichen des ersten und zweiten Qualitätskontrollprimers eine Doppelstrangstruktur gebildet wird;
(c) Unterziehen der Doppelstrangstruktur einer Amplifikationsbedingung, wodurch eine erweiterte Doppelstrangstruktur erzeugt wird und
(d) Erfassen eines Signals, das von der deaktivierbaren Markierung des ersten Qualitätskontrollprimers während der Amplifikationsreaktion erzeugt wird, um die Bildung der erweiterten Doppelstrangstruktur zu bestimmen, wobei eine Abnahme des Signals während der Amplifikationsreaktion das Erzeugen der erweiterten Doppelstrangstruktur anzeigt.

2. Verfahren nach Anspruch 1, wobei:
a. sich die deaktivierbare Markierung:
i. außerhalb des 3'-Überlappungsbereichs des ersten Qualitätskontrollprimers befindet, wobei sich die deaktivierbare Markierung optional am 5'-Terminus des ersten Qualitätskontrollprimers befindet, oder
ii. im 3'-Überlappungsbereich des ersten Qualitätskontrollprimers befindet und/oder
b. sich das Deaktivierungsmittel:
i. außerhalb des 3'-Überlappungsbereichs des zweiten Qualitätskontrollprimers befindet, wobei sich das Deaktivierungsmittel optional am 5'-Terminus des zweiten Qualitätskontrollprimers befindet, oder
ii. im 3'-Überlappungsbereich des zweiten Qualitätskontrollprimers befindet.

3. Verfahren nach Anspruch 1 oder 2, wobei:
a. der erste Qualitätskontrollprimer, der zweite Qualitätskontrollprimer oder beide umfassen:
i. ein oder mehrere modifizierte Nukleotide, wobei optional:
1. jeder der 3'-Überlappungsbereiche des ersten und zweiten Qualitätskontrollprimers ein oder mehrere modifizierte Nukleotide umfasst, und/oder
2. das eine oder die mehreren modifizierten Nukleotide einen Abstandhalter, eine a-basische Stelle, eine unmethylierte RNA-Base, ein 2'-O-methyliertes Nukleotid und eine beliebige Kombination davon umfassen, und/oder
3. es sich bei mindestens einem des einen modifizierten Nukleotids oder der mehreren modifizierten Nukleotide um ein 2'-O-methyliertes Nukleotid handelt, oder
ii. einen oder mehrere Polymerase-Stopper, wobei optional:
1. jeder der 3'-Überlappungsbereiche des ersten und zweiten Qualitätskontrollprimers einen oder mehrere Polymerase-Stopper umfasst, und/oder
2. es sich bei mindestens einem des einen oder der mehreren Polymerase-Stopper um ein 2'-O-methyliertes Nukleotid handelt, und/oder
b. der 3'-Überlappungsbereich des ersten Qualitätskontrollprimers komplementär zum 3'-Überlappungsbereich des zweiten Qualitätskontrollprimers ist, wobei optional der 3'-Überlappungsbereich des ersten Qualitätskontrollprimers vollständig komplementär zum 3'-Überlappungsbereich des zweiten Qualitätskontrollprimers ist, und/oder
c. der 3'-Überlappungsbereich des ersten Qualitätskontrollprimers und der 3'-Überlappungsbereich des zweiten Qualitätskontrollprimers dieselbe Länge aufweisen, und/oder
d. einer oder mehrere des 3'-Überlappungsbereichs des ersten Qualitätskontrollprimers und des 3'-Überlappungsbereichs des zweiten Qualitätskontrollprimers eine Länge von etwa 2 bis etwa 10 Nukleotiden aufweisen, und/oder
e. der 3'-Überlappungsbereich des ersten und zweiten Qualitätskontrollprimers 4 oder 5 Nukleotide lang ist.

4. Verfahren nach einem der Ansprüche 1-3, wobei:
a. (b) das Inkontaktbringen des ersten Qualitätskontrollprimers und des zweiten Qualitätskontrollprimers unter der Amplifikationsbedingung erfolgt und/oder
b. (d) das Erfassen des Signals, das von der deaktivierbaren Markierung des ersten Qualitätskontrollprimers während der Amplifikationsreaktion erzeugt wird, das Erfassen des Signals zu zwei oder mehr verschiedenen Zeitpunkten während der Amplifikationsreaktion umfasst und/oder
c. die Abnahme des Signals während der Amplifikationsreaktion eine Abnahme über die Zeit während der Amplifikationsreaktion umfasst, wobei die Abnahme des Signals während der Amplifikationsreaktion optional eine Abnahme umfasst über einen Zeitraum von:
i. etwa 10 Minuten während der Amplifikationsreaktion oder
ii. etwa 3 Minuten bis etwa 12 Minuten nach Beginn der Amplifikationsreaktion, und/oder
d. das Verfahren das Erfassen des Signals der deaktivierbaren Markierung des ersten Qualitätskontrollprimers vor der Amplifikationsreaktion, nach der Amplifikationsreaktion oder beides umfasst.

5. Verfahren nach einem der Ansprüche 1-4, wobei:
a. es sich bei der Amplifikationsreaktion um eine Echtzeit-Amplifikationsreaktion handelt und/oder
b. die Amplifikationsreaktion:
i. eine PCR-Reaktion ist oder
ii. eine oder mehrere der Folgenden umfasst: Archaea-Polymerase-Amplifikation (APA), Loop-vermittelte isotherme Amplifikation (LAMP), Helikase-abhängige Amplifikation (HDA), Rekombinase-Polymerase-Amplifikation (RPA), Strangverdrängungsamplifikation (SDA), Nukleinsäuresequenz-basierte Amplifikation (NASBA), transkriptionsvermittelte Amplifikation (TMA), Nicking-Enzym-Amplifikationsreaktion (NEAR), Rolling-Circle-Amplifikation (RCA), multiple Verdrängungsamplifikation (MDA), Ramifikation (RAM), zirkuläre Helikase-abhängige Amplifikation (cHDA), isotherme Einzelprimer-Amplifikation (SPIA), signalvermittelte Amplifikation der RNA-Technologie (SMART), selbsttragende Sequenzreplikation (3SR), exponentielle Genomamplifikationsreaktion (GEAR) und isotherme multiple Verdrängungsamplifikation (IMDA); oder
iii. eine isotherme Amplifikationsreaktion ist, wobei optional:
1. die Amplifikationsbedingung ein oder mehrere Enzyme mit hyperthermophiler Polymeraseaktivität, dNTPs und einem Pufferungsmittel umfasst, wobei das Enzym mit hyperthermophiler Polymeraseaktivität ferner optional eine Aminosäuresequenz aufweist, die zu mindestens etwa 90 % mit der Aminosäuresequenz von SEQ ID NO: 1 oder einem funktionellen Fragment davon identisch ist, und das Enzym mit hyperthermophiler Polymeraseaktivität optional die Aminosäuresequenz von SEQ ID NO: 1 umfasst, und/oder
2. die isotherme Amplifikationsreaktion eine konstante Temperatur von etwa 30 °C bis etwa 72 °C umfasst, und optional die isotherme Amplifikationsreaktion eine konstante Temperatur von etwa 67 °C umfasst, und/oder
3. die isotherme Amplifikationsreaktion über einen Zeitraum von etwa 5 Minuten bis etwa 60 Minuten durchgeführt wird, und/oder
4. die isotherme Amplifikationsreaktion unter isothermer Amplifikationsbedingung durchgeführt wird, die frei von Helikase, einzelsträngigen Bindungsproteinen, Spaltmitteln und Rekombinasen sind.

6. Verfahren nach einem der Ansprüche 1-5, wobei:
a. das Erfassen des Signals, das von der deaktivierbaren Markierung des ersten Qualitätskontrollprimers erzeugt wird, nicht die Verwendung einer Sonde umfasst, und/oder
b. (c) das Unterziehen der Doppelstrangstruktur der Amplifikationsbedingung das Unterziehen einer Ziel-Nukleinsäure und eines oder mehrerer zusätzlicher Primer und/oder einer oder mehrerer für die Ziel-Nukleinsäure spezifischer Sonden der Amplifikationsbedingung umfasst, wobei optional:
i. der erste Qualitätskontrollprimer unter der Amplifikationsbedingung nicht mit der Ziel-Nukleinsäure hybridisiert und/oder
ii. der zweite Qualitätskontrollprimer unter der Amplifikationsbedingung nicht mit der Ziel-Nukleinsäure hybridisiert.

7. Verfahren nach einem der Ansprüche 1-6, wobei:
a. das Verfahren nicht die Verwendung einer Muster-Nukleinsäure umfasst, die zur Hybridisierung mit dem ersten Qualitätskontrollprimer fähig ist, und/oder
a. das Verfahren nicht die Verwendung einer Muster-Nukleinsäure umfasst, die zur Hybridisierung mit dem zweiten Qualitätskontrollprimer fähig ist.

8. Kit zur Überprüfung einer Amplifikationsreaktion, umfassend
einen ersten Qualitätskontrollprimer und einen zweiten Qualitätskontrollprimer, die jeweils einen 3'-Überlappungsbereich umfassen, der in der Lage ist, sie miteinander zu hybridisieren, wobei der erste Qualitätskontrollprimer und der zweite Qualitätskontrollprimer jeweils nicht länger als 35 Nukleotide sind, wobei der erste Qualitätskontrollprimer eine deaktivierbare Markierung umfasst und wobei der zweite Qualitätskontrollprimer ein Deaktivierungsmittel umfasst.

9. Kit nach Anspruch 8, wobei:
a. es sich bei der deaktivierbaren Markierung um einen Fluorophor handelt, und/oder
b. die deaktivierbare Markierung sich:
i. außerhalb des 3'-Überlappungsbereichs des ersten Qualitätskontrollprimers befindet, wobei sich die deaktivierbare Markierung optional am 5'-Terminus des ersten Qualitätskontrollprimers befindet, oder
ii. die deaktivierbare Markierung sich im 3'-Überlappungsbereich des ersten Qualitätskontrollprimers befindet und/oder
c. das Deaktivierungsmittel sich:
i. außerhalb des 3'-Überlappungsbereichs des zweiten Qualitätskontrollprimers befindet, wobei sich das Deaktivierungsmittel optional am 5'-Terminus des zweiten Qualitätskontrollprimers befindet, oder
ii. das Deaktivierungsmittel sich im 3'-Überlappungsbereich des zweiten Qualitätskontrollprimers befindet.

10. Kit nach einem der Ansprüche 8 oder 9, wobei:
a. der erste Qualitätskontrollprimer, der zweite Qualitätskontrollprimer oder beide umfassen:
i. ein oder mehrere modifizierte Nukleotide, wobei optional:
1. jeder der 3'-Überlappungsbereiche des ersten und zweiten Qualitätskontrollprimers ein oder mehrere modifizierte Nukleotide umfasst, und/oder
2. das eine modifizierte Nukleotid oder die mehreren modifizierten Nukleotide einen Abstandhalter, eine a-basische Stelle, eine unmethylierte RNA-Base, ein 2'-O-methyliertes Nukleotid und eine beliebige Kombination davon umfassen, oder
ii. einen oder mehrere Polymerase-Stopper, wobei optional:
1. jeder der 3'-Überlappungsbereiche des ersten und zweiten Qualitätskontrollprimers einen oder mehrere Polymerase-Stopper umfasst, und/oder
2. es sich bei mindestens einem des einen oder der mehreren Polymerase-Stopper um ein 2'-O-methyliertes Nukleotid handelt, und/oder
b. der 3'-Überlappungsbereich des ersten Qualitätskontrollprimers komplementär zum 3'-Überlappungsbereich des zweiten Qualitätskontrollprimers ist, wobei optional der 3'-Überlappungsbereich des ersten Qualitätskontrollprimers vollständig komplementär zum 3'-Überlappungsbereich des zweiten Qualitätskontrollprimers ist, und/oder
c. der 3'-Überlappungsbereich des ersten Qualitätskontrollprimers und der 3'-Überlappungsbereich des zweiten Qualitätskontrollprimers dieselbe Länge aufweisen, und/oder
d. einer oder mehrere des 3'-Überlappungsbereichs des ersten Qualitätskontrollprimers und des 3'-Überlappungsbereichs des zweiten Qualitätskontrollprimers eine Länge von etwa 2 bis etwa 10 Nukleotiden aufweisen, und/oder
e. der 3'-Überlappungsbereich des ersten und zweiten Qualitätskontrollprimers 4 oder 5 Nukleotide lang ist.

11. Kit nach einem der Ansprüche 8 bis 10, umfassend ein Enzym oder mehrere Enzyme mit hyperthermophiler Polymeraseaktivität, dNTPs und einem Pufferungsmittel, wobei das Enzym mit hyperthermophiler Polymeraseaktivität optional eine Aminosäuresequenz aufweist, die zu mindestens etwa 90 % der Aminosäuresequenz von SEQ ID NO: 1 oder einem funktionellen Fragment davon entspricht, wobei das Enzym mit hyperthermophiler Polymeraseaktivität optional die Aminosäuresequenz von SEQ ID NO: 1 umfasst.

12. Kit nach einem der Ansprüche 8 bis 11, wobei:
a. der erste Qualitätskontrollprimer und der zweite Qualitätskontrollprimer in lyophilisierter oder gefriergetrockneter Form vorliegen, wobei optional das eine oder die mehreren Enzyme mit hyperthermophiler Polymeraseaktivität, dNTPs und einem Pufferungsmittel in lyophilisierter oder gefriergetrockneter Form vorliegen, und/oder
b. das Kit ferner einen oder mehrere zusätzliche Primer und/oder eine oder mehrere Sonden umfasst, die für eine Ziel-Nukleinsäure spezifisch sind.

13. Reaktionsgemisch, umfassend
einen ersten Qualitätskontrollprimer und einen zweiten Qualitätskontrollprimer, die jeweils einen 3'-Überlappungsbereich umfassen, der in der Lage ist, sie miteinander zu hybridisieren, wobei der erste Qualitätskontrollprimer und der zweite Qualitätskontrollprimer jeweils nicht länger als 35 Nukleotide ist, wobei der erste Qualitätskontrollprimer eine deaktivierbare Markierung umfasst und wobei der zweite Qualitätskontrollprimer ein Deaktivierungsmittel umfasst,
eine Ziel-Nukleinsäure und
einen oder mehrere zusätzliche Primer und/oder eine oder mehrere Sonden, die für eine Ziel-Nukleinsäure spezifisch sind.

14. Reaktionsgemisch nach Anspruch 13, wobei:
a. das Reaktionsgemisch eine Doppelstrangstruktur umfasst, die durch Hybridisierung zwischen den 3'-Überlappungsbereichen des ersten und zweiten Qualitätskontrollprimers gebildet wird, und/oder
b. das Reaktionsgemisch ein oder mehrere Enzyme mit Polymeraseaktivität, dNTPs und einem Pufferungsmittel umfasst; und es sich bei dem Enzym optional um ein Enzym mit hyperthermophiler Polymeraseaktivität handelt, wobei das Enzym mit hyperthermophiler Polymeraseaktivität optional eine Aminosäuresequenz aufweist, die zu mindestens etwa 90 % mit der Aminosäuresequenz von SEQ ID NO: 1 oder einem funktionellen Fragment davon identisch ist, und das Enzym mit hyperthermophiler Polymeraseaktivität optional die Aminosäuresequenz von SEQ ID NO: 1 umfasst.

15. Reaktionsgemisch nach einem der Ansprüche 13 oder 14, wobei:
a. der erste Qualitätskontrollprimer und der zweite Qualitätskontrollprimer nicht zur Hybridisierung mit der Ziel-Nukleinsäure in der Lage sind, und/oder
b. der eine oder die mehreren zusätzlichen Primer und/oder die eine oder die mehreren Sonden nicht in der Lage sind, mit dem ersten Qualitätskontrollprimer, dem zweiten Qualitätskontrollprimer oder beiden zu hybridisieren.

## Revendications

1. Procédé de surveillance d'une réaction d'amplification, comprenant
(a) la fourniture d'une première amorce de contrôle de qualité et d'une deuxième amorce de contrôle de qualité comprenant chacune une région de chevauchement 3' pouvant s'hybrider l'une à l'autre, chacune de la première amorce de contrôle de qualité et la deuxième amorce de contrôle de qualité ayant une longueur inférieure ou égale à 35 nucléotides, la première amorce de contrôle de qualité comprenant un marqueur quenchable, le marqueur quenchable étant un fluorophore, et la deuxième amorce de contrôle de qualité comprenant un quencher ;
(b) la mise en contact de la première amorce de contrôle de qualité et de la deuxième amorce de contrôle de qualité, formant ainsi un double brin par hybridation entre les régions de chevauchement 3' des première et deuxième amorces de contrôle de qualité ;
(c) la soumission du double brin à une condition d'amplification, générant ainsi un double brin étendu ; et
(d) la détection d'un signal généré à partir du marqueur quenchable de la première amorce de contrôle de qualité pendant la réaction d'amplification pour déterminer la génération du double brin étendu, une diminution du signal pendant la réaction d'amplification indiquant la génération du double brin étendu.

2. Procédé selon la revendication 1, dans lequel :
a. le marqueur quenchable est :
i. à l'extérieur de la région de chevauchement 3' de la première amorce de contrôle de qualité, facultativement, le marqueur quenchable étant situé à l'extrémité 5' de la première amorce de contrôle de qualité ; ou
ii. dans la région de chevauchement 3' de la première amorce de contrôle de qualité ; et/ou
b. le quencher est :
i. à l'extérieur de la région de chevauchement 3' de la deuxième amorce de contrôle de qualité, facultativement, le quencher étant situé à l'extrémité 5' de la deuxième amorce de contrôle de qualité ; ou
ii. dans la région de chevauchement 3' de la deuxième amorce de contrôle de qualité.

3. Procédé selon l'une quelconque des revendications 1 à 2, dans lequel :
a. la première amorce de contrôle de qualité, la deuxième amorce de contrôle de qualité, ou les deux, comprend :
i. un ou plusieurs nucléotides modifiés, facultativement dans lequel :
1. chacune des régions de chevauchement 3' des première et deuxième amorces de contrôle de qualité comprend un ou plusieurs nucléotides modifiés ; et/ou
2. le ou les nucléotides modifiés comprennent un espaceur, un site abasique, une base d'ARN non méthylée, un nucléotide 2'-O-méthylé, et une combinaison quelconque de ceux-ci ; et/ou
3. au moins un des nucléotides modifiés est un nucléotide 2'-O-méthylé ; ou
ii. un ou plusieurs bloqueurs de polymérase, facultativement dans lequel :
1. chacune des régions de chevauchement 3' des première et deuxième amorces de contrôle de qualité comprend un ou plusieurs bloqueurs de polymérase ; et/ou
2. au moins un des bloqueurs de polymérase est un nucléotide 2'-O-méthylé ; et/ou
b. la région de chevauchement 3' de la première amorce de contrôle de qualité est complémentaire de la région de chevauchement 3' de la deuxième amorce de contrôle de qualité, facultativement, la région de chevauchement 3' de la première amorce de contrôle de qualité étant totalement complémentaire de la région de chevauchement 3' de la deuxième amorce de contrôle de qualité ; et/ou
c. la région de chevauchement 3' de la première amorce de contrôle de qualité et la région de chevauchement 3' de la deuxième amorce de contrôle de qualité ont la même longueur ; et/ou
d. une ou plusieurs parmi la région de chevauchement 3' de la première amorce de contrôle de qualité et la région de chevauchement 3' de la deuxième amorce de contrôle de qualité a une longueur d'environ 2 à environ 10 nucléotides ; et/ou
e. la région de chevauchement 3' des première et deuxième amorces de contrôle de qualité a une longueur de 4 ou 5 nucléotides.

4. Procédé selon l'une quelconque des revendications 1 à 3, dans lequel :
a. (b) la mise en contact de la première amorce de contrôle de qualité et de la deuxième amorce de contrôle de qualité est effectuée dans les conditions d'amplification ; et/ou
b. (d) la détection du signal généré à partir du marqueur quenchable de la première amorce de contrôle de qualité pendant la réaction d'amplification comprend la détection du signal à au moins deux instants différents pendant la réaction d'amplification ; et/ou
c. la diminution du signal pendant la réaction d'amplification comprend une diminution au cours du temps pendant la réaction d'amplification, facultativement, la diminution du signal pendant la réaction d'amplification comprend une diminution sur une période de temps de :
i. environ 10 minutes pendant la réaction d'amplification ; ou
ii. environ 3 minutes à environ 12 minutes à partir du début de la réaction d'amplification ; et/ou
d. le procédé comprend la détection du signal du marqueur quenchable de la première amorce de contrôle de qualité avant la réaction d'amplification, après la réaction d'amplification, ou les deux.

5. Procédé selon l'une quelconque des revendications 1 à 4, dans lequel :
a. la réaction d'amplification est une réaction d'amplification en temps réel ; et/ou
b. la réaction d'amplification :
i. est une réaction de PCR ; ou
ii. comprend un ou plusieurs des éléments suivants : amplification par polymérase archéale (APA), amplification isotherme à médiation par boucle (LAMP), amplification dépendante de l'hélicase (HDA), amplification par recombinase polymérase (RPA), amplification par déplacement de brin (SDA), amplification basée sur une séquence d'acide nucléique (NASBA), amplification médiée par transcription (TMA), réaction d'amplification par enzyme de coupure (NEAR), amplification par cercle roulant (RCA), amplification par déplacement multiple (MDA), amplification par ramification (RAM), amplification dépendante de l'hélicase circulaire (cHDA), amplification isotherme à amorce unique (SPIA), technologie d'amplification de l'ARN médiée par signal (SMART), réplication de séquence auto-entretenue (3SR), réaction d'amplification exponentielle du génome (GEAR) et amplification isotherme par déplacement multiple (IMDA) ; ou
iii. est une réaction d'amplification isotherme, facultativement dans lequel :
1. les conditions d'amplification comprennent un ou plusieurs éléments parmi une enzyme ayant une activité polymérase hyperthermophile, des dNTP, et un agent tampon, en outre, facultativement, l'enzyme ayant une activité polymérase hyperthermophile ayant une séquence d'acides aminés qui présente au moins environ 90 % d'identité avec la séquence d'acides aminés de SEQ ID NO : 1 ou un fragment fonctionnel de celle-ci, et facultativement, l'enzyme ayant une activité polymérase hyperthermophile comprenant la séquence d'acides aminés de SEQ ID NO : 1 ; et/ou
2. la réaction d'amplification isotherme comprend une température constante d'environ 30 °C à environ 72 °C, et facultativement, la réaction d'amplification isotherme comprend une température constante d'environ 67 °C ; et/ou
3. la réaction d'amplification isotherme est réalisée pendant une durée d'environ 5 minutes à environ 60 minutes ; et/ou
4. la réaction d'amplification isotherme est réalisée dans des conditions d'amplification isotherme sans hélicase, sans protéine de liaison simple brin, sans agent de clivage et sans recombinase.

6. Procédé selon l'une quelconque des revendications 1 à 5, dans lequel :
a. la détection du signal généré à partir du marqueur quenchable de la première amorce de contrôle de qualité ne comprend l'utilisation d'aucune sonde ; et/ou
b. (c) la soumission du double brin aux conditions d'amplification comprend la soumission d'un acide nucléique cible et d'une ou plusieurs amorces supplémentaires et/ou une ou plusieurs sondes spécifiques de l'acide nucléique cible aux conditions d'amplification, facultativement dans lequel :
i. la première amorce de contrôle de qualité ne s'hybride pas à l'acide nucléique cible dans les conditions d'amplification ; et/ou
ii. la deuxième amorce de contrôle de qualité ne s'hybride pas à l'acide nucléique cible dans les conditions d'amplification.

7. Procédé selon l'une quelconque des revendications 1 à 6, dans lequel :
a. le procédé ne comprend pas l'utilisation d'une matrice d'acide nucléique pouvant s'hybrider à la première amorce de contrôle de qualité ; et/ou
b. le procédé ne comprend pas l'utilisation d'une matrice d'acide nucléique pouvant s'hybrider à la deuxième amorce de contrôle de qualité.

8. Kit pour surveiller une réaction d'amplification, comprenant
une première amorce de contrôle de qualité et une deuxième amorce de contrôle de qualité comprenant chacune une région de chevauchement en 3' capable de s'hybrider l'une à l'autre, chacune de la première amorce de contrôle de qualité et de la deuxième amorce de contrôle de qualité ayant une longueur inférieure ou égale à 35 nucléotides, la première amorce de contrôle de qualité comprenant un marqueur quenchable, et la deuxième amorce de contrôle de qualité comprenant un quencher.

9. Kit selon la revendication 8, dans lequel :
a. le marqueur quenchable est un fluorophore ; et/ou
b. le marqueur quenchable est :
i. à l'extérieur de la région de chevauchement 3' de la première amorce de contrôle de qualité, facultativement, le marqueur quenchable étant situé à l'extrémité 5' de la première amorce de contrôle de qualité ; ou
ii. le marqueur quenchable est situé dans la région de chevauchement 3' de la première amorce de contrôle de qualité ; et/ou
c. le quencher est :
i. à l'extérieur de la région de chevauchement 3' de la deuxième amorce de contrôle de qualité, facultativement, le quencher étant situé à l'extrémité 5' de la deuxième amorce de contrôle de qualité ; ou
ii. le quencher est situé dans la région de chevauchement 3' de la deuxième amorce de contrôle de qualité.

10. Kit selon l'une quelconque des revendications 8 ou 9, dans lequel :
a. la première amorce de contrôle de qualité, la deuxième amorce de contrôle de qualité, ou les deux, comprend :
i. un ou plusieurs nucléotides modifiés, facultativement dans lequel :
1. chacune des régions de chevauchement 3' des première et deuxième amorces de contrôle de qualité comprend un ou plusieurs nucléotides modifiés ; et/ou
2. les un ou plusieurs nucléotides modifiés comprennent un espaceur, un site abasique, une base d'ARN non méthylée, un nucléotide 2'-O-méthylé, et une combinaison quelconque de ceux-ci ; ou
ii. un ou plusieurs bloqueurs de polymérase, facultativement dans lequel :
1. chacune des régions de chevauchement 3' des première et deuxième amorces de contrôle de qualité comprend un ou plusieurs bloqueurs de polymérase ; et/ou
2. au moins un des bloqueurs de polymérase est un nucléotide 2'-O-méthylé ; et/ou
b. la région de chevauchement 3' de la première amorce de contrôle de qualité est complémentaire de la région de chevauchement 3' de la deuxième amorce de contrôle de qualité, facultativement, la région de chevauchement 3' de la première amorce de contrôle de qualité étant totalement complémentaire de la région de chevauchement 3' de la deuxième amorce de contrôle de qualité ; et/ou
c. la région de chevauchement 3' de la première amorce de contrôle de qualité et la région de chevauchement 3' de la deuxième amorce de contrôle de qualité ont la même longueur ; et/ou
d. une ou plusieurs parmi la région de chevauchement 3' de la première amorce de contrôle de qualité et la région de chevauchement 3' de la deuxième amorce de contrôle de qualité a une longueur d'environ 2 à environ 10 nucléotides ; et/ou
e. la région de chevauchement 3' des première et deuxième amorces de contrôle de qualité a une longueur de 4 ou 5 nucléotides.

11. Kit selon l'une quelconque des revendications 8 à 10, comprenant un ou plusieurs éléments parmi une enzyme ayant une activité polymérase hyperthermophile, des dNTP, et un agent tampon, en outre, facultativement, l'enzyme ayant une activité polymérase hyperthermophile ayant une séquence d'acides aminés qui présente au moins environ 90 % d'identité avec la séquence d'acides aminés de SEQ ID NO : 1 ou un fragment fonctionnel de celle-ci, facultativement, l'enzyme ayant une activité polymérase hyperthermophile comprenant la séquence d'acides aminés de SEQ ID NO : 1.

12. Kit selon l'une quelconque des revendications 8 à 11, dans lequel :
a. la première amorce de contrôle de qualité et la deuxième amorce de contrôle de qualité sont sous une forme lyophilisée, facultativement, la ou les enzymes ayant une activité polymérase hyperthermophile, les dNTP et un agent tampon étant sous une forme lyophilisée ; et/ou
b. le kit comprend en outre une ou plusieurs amorces supplémentaires et/ou une ou plusieurs sondes spécifiques d'un acide nucléique cible.

13. Mélange réactionnel, comprenant
une première amorce de contrôle de qualité et une deuxième amorce de contrôle de qualité comprenant chacune une région de chevauchement en 3' capable de s'hybrider l'une à l'autre, chacune de la première amorce de contrôle de qualité et de la deuxième amorce de contrôle de qualité ayant une longueur inférieure ou égale à 35 nucléotides, la première amorce de contrôle de qualité comprenant un marqueur quenchable, et la deuxième amorce de contrôle de qualité comprenant un quencher;
un acide nucléique cible ; et
une ou plusieurs amorces supplémentaires et/ou une ou plusieurs sondes spécifiques de l'acide nucléique cible.

14. Mélange réactionnel selon la revendication 13, dans lequel :
a. le mélange réactionnel comprend un double brin formé par hybridation entre les régions de chevauchement 3' des première et deuxième amorces de contrôle de qualité ; et/ou
b. le mélange réactionnel comprend un ou plusieurs éléments parmi une enzyme ayant une activité polymérase hyperthermophile, des dNTP, et un agent tampon ; et facultativement, l'enzyme est une enzyme ayant une activité polymérase hyperthermophile, facultativement, l'enzyme ayant une activité polymérase hyperthermophile ayant une séquence d'acides aminés qui présente au moins environ 90 % d'identité avec la séquence d'acides aminés de SEQ ID NO : 1 ou un fragment fonctionnel de celle-ci, et facultativement, l'enzyme ayant une activité polymérase hyperthermophile comprenant la séquence d'acides aminés de SEQ ID NO : 1.

15. Mélange réactionnel selon l'une quelconque des revendications 13 à 14, dans lequel :
a. la première amorce de contrôle de qualité et la deuxième amorce de contrôle de qualité ne peuvent pas s'hybrider à l'acide nucléique cible ; et/ou
b. la ou les amorces supplémentaires et/ou la ou les sondes ne peuvent pas s'hybrider à la première amorce de contrôle de qualité, à la deuxième amorce de contrôle de qualité, ou aux deux.
